(19) Europäisches Patentamt
European Patent Office
Office européen des brevets

(11) EP 3 778 914 A1

(12) **EUROPEAN PATENT APPLICATION**
published in accordance with Art. 153(4) EPC

(43) Date of publication:
**17.02.2021 Bulletin 2021/07**

(21) Application number: **19775179.5**

(22) Date of filing: **28.03.2019**

(51) Int Cl.:
***C12P 19/34*** *(2006.01)* ***C12N 15/10*** *(2006.01)*
***C12N 15/113*** *(2010.01)*

(86) International application number:
**PCT/JP2019/013631**

(87) International publication number:
**WO 2019/189591 (03.10.2019 Gazette 2019/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.03.2018 JP 2018069560**

(71) Applicant: **Sumitomo Chemical Company, Ltd.**
**Chuo-ku**
**Tokyo 104-8260 (JP)**

(72) Inventor: **SAKATA Akihiro**
**Osaka-shi, Osaka 555-0021 (JP)**

(74) Representative: **Arends, William Gerrit**
**Marks & Clerk LLP**
**15 Fetter Lane**
**London EC4A 1BW (GB)**

(54) **METHOD FOR PRODUCING SINGLE-STRAND RNA**

(57) A method for producing a single-strand RNA includes a step of causing an RNA ligase which is classified as EC 6.5.1.3 defined by the International Union of Biochemistry and Molecular Biology as an enzyme number and has a nick repair activity to act on a first single-strand RNA and a second single-strand RNA, which have a phosphate group at the 5' end, and a third single-strand RNA having a hydroxyl group at the 5' end to link the first single-strand RNA, the second single-strand RNA, and the third single-strand RNA.

FIG. 1

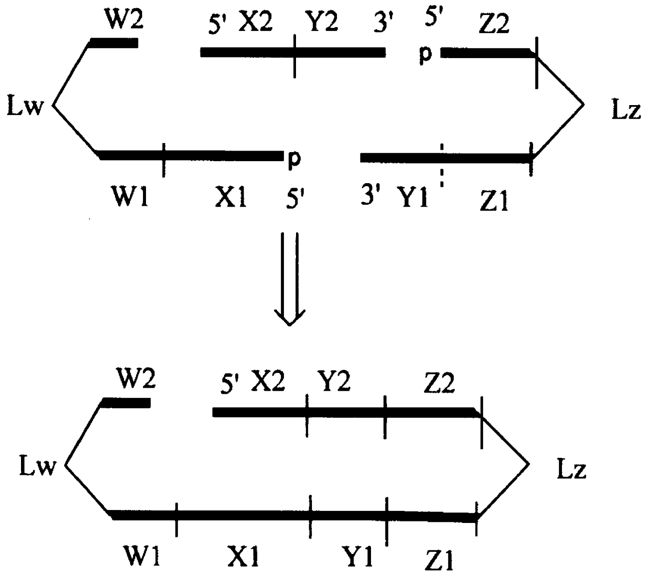


EP 3 778 914 A1

## Description

[Technical Field]

The present invention relates to a method for producing a single-strand RNA.

Priority is claimed on Japanese Patent Application No. 2018-069560, filed March 30, 2018, the content of which is incorporated herein by reference.

[Background Art]

Patent Document 1 reports a nucleic acid compound as a nucleic acid molecule capable of suppressing expression of a gene. As such a nucleic acid, a nucleic acid having an amino acid structure in a linker portion of the nucleic acid compound is also known. Moreover, these nucleic acid compounds are less likely to be decomposed in a living body, have high biostability, and have a characteristic of avoiding an immune response.

Since a nucleic acid compound having such an excellent characteristic has a long strand length of about 50 bases, a simpler method is required as a synthetic method.

[Citation List]

[Patent Literature]

[Patent Document 1]

PCT International Publication No. WO2012/017919

[Summary of Invention]

[Technical Problem]

An object of the present invention is to provide a simple production method for a single-strand RNA.

[Solution to Problem]

The present inventors have conducted intensive research to achieve the object, and as a result, have found that by synthesizing three RNAs having a length of about 20 bases and then performing ligation with a certain type of RNA ligase, a single-strand RNA having a length of 50 bases or more can be easily produced.

Furthermore, it has been found that by ligating, with a certain type of RNA ligase, a single-strand RNA consisting of two RNAs, that is, first and second RNAs among the three single-strand RNAs, to a third RNA, a desired single-strand RNA having a length of 50 bases or more can be easily produced.

The present invention has been completed through further examination based on these findings, and provides the following method for producing a single-strand RNA.

[1] A method for producing a single-strand RNA, including a step of causing an RNA ligase which is classified as EC 6.5.1.3 defined by the International Union of Biochemistry and Molecular Biology as an enzyme number and has a nick repair activity to act on a first single-strand RNA and a second single-strand RNA, which have a phosphate group at the 5' end, and a third single-strand RNA having a hydroxyl group at the 5' end to link the first single-strand RNA, the second single-strand RNA, and the third single-strand RNA,

in which a) the first single-strand RNA is a single-strand RNA consisting of an X1 region, a W1 region, an Lw linker region, and a W2 region in order from the 5' end,
b) the second single-strand RNA is a single-strand RNA consisting of a Z2 region, an Lz linker region, a Z1 region, and a Y1 region in order from the 5' end,
c) the third single-strand RNA is a single-strand RNA consisting of an X2 region and a Y2 region in order from the 5' end,
d) the X1 region and the X2 region have nucleotide sequences which are complementary to each other and have the same number of nucleotides of two or more,
e) the Y1 region and the Y2 region have nucleotide sequences which are complementary to each other and have the same number of nucleotides of two or more,

f) the Z1 region and the Z2 region have nucleotide sequences which are complementary to each other and have the same number of nucleotides of one or more,

g) the W1 region and the W2 region each independently have any number of nucleotide sequences,

h) the Lz linker region and the Lw linker region are each independently a linker region having an atomic group derived from an amino acid, and

i) a single-strand RNA generated by linking the first single-strand RNA, the second single-strand RNA, and the third single-strand RNA is a linked single-strand RNA consisting of the X2 region, the Y2 region, the Z2 region, the Lz linker region, the Z1 region, the Y1 region, the X1 region, the W1 region, the Lw linker region, and the W2 region in order from the 5' end.

[2] The production method according to [1], in which the Lz linker region is a divalent group represented by Formula (I).

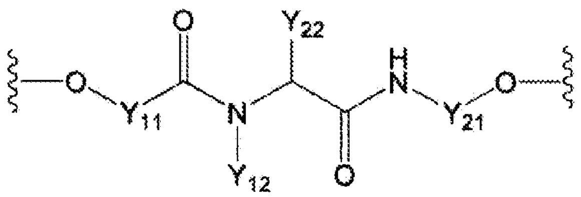

(I)

In the formula, $Y_{11}$ and $Y_{21}$ each independently represent an alkylene group having 1 to 20 carbon atoms, and $Y_{12}$ and $Y_{22}$ each independently represent a hydrogen atom or an alkyl group which may be substituted with an amino group, or $Y_{12}$ and $Y_{22}$ are bonded to each other at their terminals to represent an alkylene group having 3 or 4 carbon atoms,

a terminal oxygen atom bonded to $Y_{11}$ is bonded to a phosphorus atom of a phosphate ester of a terminal nucleotide in any one of the Z1 region and the Z2 region, and

a terminal oxygen atom bonded to $Y_{21}$ is bonded to a phosphorus atom of a phosphate ester of a terminal nucleotide in the other of the Z2 region and the Z1 region, which is not bonded to Y11.

[3] The production method according to [2], in which the Lz linker region is a divalent group represented by Formula (1), and the Lw linker region is a divalent group represented by Formula (I').

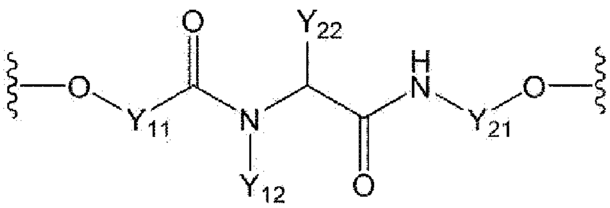

(I)

In the formula, $Y_{11}$ and $Y_{21}$ each independently represent an alkylene group having 1 to 20 carbon atoms, and $Y_{12}$ and $Y_{22}$ each independently represent a hydrogen atom or an alkyl group which may be substituted with an amino group, or $Y_{12}$ and $Y_{22}$ are bonded to each other at their terminals to represent an alkylene group having 3 or 4 carbon atoms,

a terminal oxygen atom bonded to $Y_{11}$ is bonded to a phosphorus atom of a phosphate ester of a terminal nucleotide in any one region of the Z1 region and the Z2 region, and

a terminal oxygen atom bonded to $Y_{21}$ is bonded to a phosphorus atom of a phosphate ester of a terminal nucleotide in the other region of the Z2 region and the Z1 region, which is not bonded to Y11.

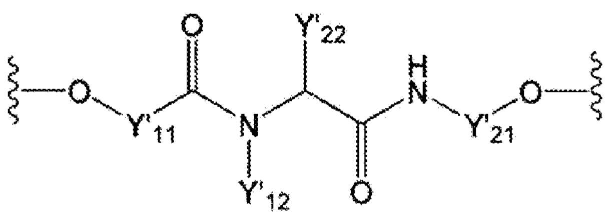

(I')

In the formula, $Y'_{11}$ and $Y'_{21}$ each independently represent an alkylene group having 1 to 20 carbon atoms, and $Y'_{12}$ and $Y'_{22}$ each independently represent a hydrogen atom or an alkyl group which may be substituted with an

amino group, or $Y'_{12}$ and $Y'_{22}$ are bonded to each other at their terminals to represent an alkylene group having 3 or 4 carbon atoms,

a terminal oxygen atom bonded to $Y'_{11}$ is bonded to a phosphorus atom of a phosphate ester of a terminal nucleotide in any one of the W1 region and the W2 region, and

a terminal oxygen atom bonded to $Y'_{21}$ is bonded to a phosphorus atom of a phosphate ester of a terminal nucleotide in the other of the W2 region and the W1 region, which is not bonded to $Y'_{11}$.

[4] The production method according to [2] or [3], in which the divalent group represented by Formula (I) is a divalent group represented by Formula (II-A) or (II-B).

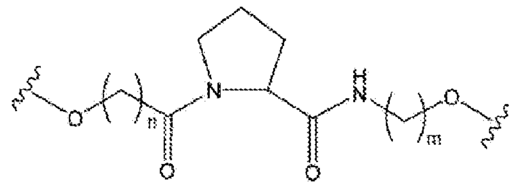

(II-A)

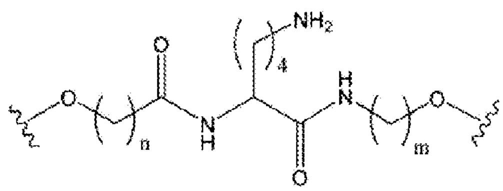

(II-B)

In the formulae, n and m each independently represent any integer of 1 to 20.

[5] The production method according to [3] or [4], in which the divalent group represented by Formula (I') is a divalent group represented by Formula (II-A) or (II-B).

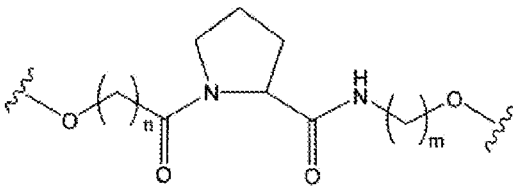

(II-A)

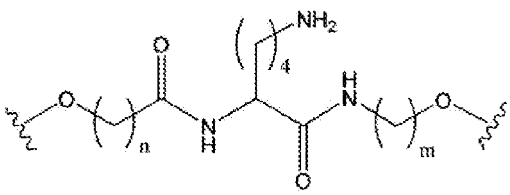

(II-B)

In the formulae, n and m each independently represent any integer of 1 to 20.

[6] The production method according to any one of [1] to [5], in which a nucleotide sequence that suppresses expression of a gene is included in at least one of a region consisting of the W1 region, the X1 region, the Y1 region, and the Z1 region, and a region consisting of the X2 region, the Y2 region, and the Z2 region.

[7] The production method according to any one of [1] to [6], in which a single-strand RNA which is generated from the first single-strand RNA and the second RNA strand, and consists of the Z2 region, the Lz linker region, the Z1 region, the Y1 region, the X1 region, the W1 region, the Lw linker region, and the W2 region in order from the 5' end is reacted with the third RNA.

[8] The production method according to any one of [1] to [6], in which a single-strand RNA which is generated from the second single-strand RNA and the third RNA strand, and consists of the X2 region, the Y2 region, the Z2 region, the Lz linker region, the Z1 region, and the Y1 region in order from the 5' end is reacted with the first RNA.

[9] A method for producing a single-strand RNA, including a step of causing an RNA ligase which is classified as EC 6.5.1.3 defined by the International Union of Biochemistry and Molecular Biology as an enzyme number and has a nick repair activity to act on a first single-strand RNA and a second single-strand RNA which have a phosphate group at the 5' end to link the first single-strand RNA and the second single-strand RNA,

in which a) the first single-strand RNA is a single-strand RNA consisting of a Y2a region, an Lz linker region, a Y1a region, an X1a region, a W1a region, an Lw linker region, and a W2a region in order from the 5' end,
b) the second single-strand RNA is a single-strand RNA consisting of an X2a region,
c) the X1a region and the X2a region have nucleotide sequences which are complementary to each other and have the same number of nucleotides of four or more,
d) the Y1a region and the Y2a region have nucleotide sequences which are complementary to each other and have the same number of nucleotides of one or more,
e) the W1a region and the W2a region each independently have any number of nucleotide sequences,
f) the Lw linker region and the Lz linker region are each independently a linker region having an atomic group derived from an amino acid, and
g) a single-strand RNA generated by linking the first single-strand RNA and the second single-strand RNA is a linked single-strand RNA consisting of the X2a region, the Y2a region, the Lz linker region, the Y1a region, the X1a region, the W1a region, the Lw linker region, and the W2a region in order from the 5' end.

[10] The production method according to [9], in which the Lz linker region is a divalent group represented by Formula (I).

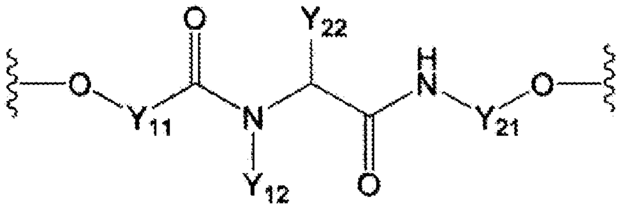

(I)

In the formula, $Y_{11}$ and $Y_{21}$ each independently represent an alkylene group having 1 to 20 carbon atoms, and $Y_{12}$ and $Y_{22}$ each independently represent a hydrogen atom or an alkyl group which may be substituted with an amino group, or $Y_{12}$ and $Y_{22}$ are bonded to each other at their terminals to represent an alkylene group having 3 or 4 carbon atoms,
a terminal oxygen atom bonded to $Y_{11}$ is bonded to a phosphorus atom of a phosphate ester of a terminal nucleotide in any one of the Y1a region and the Y2a region, and
a terminal oxygen atom bonded to $Y_{21}$ is bonded to a phosphorus atom of a phosphate ester of a terminal nucleotide in the other of the Y1a region and the Y2a region, which is not bonded to $Y_{11}$.
[11] The production method according to [9], in which the Lz linker region is a divalent group represented by Formula (I), and the Lw linker region is a divalent group represented by Formula (I').

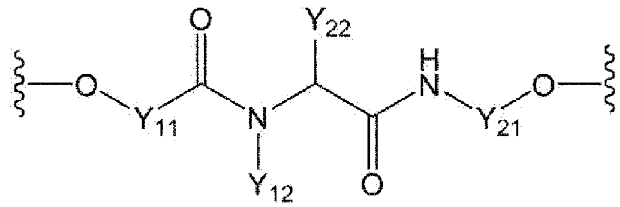

(I)

In the formula, $Y_{11}$ and $Y_{21}$ each independently represent an alkylene group having 1 to 20 carbon atoms, and $Y_{12}$ and $Y_{22}$ each independently represent a hydrogen atom or an alkyl group which may be substituted with an amino group, or $Y_{12}$ and $Y_{22}$ are bonded to each other at their terminals to represent an alkylene group having 3 or 4 carbon atoms,
a terminal oxygen atom bonded to $Y_{11}$ is bonded to a phosphorus atom of a phosphate ester of a terminal nucleotide in any one region of the Y1a region and the Y2a region, and
a terminal oxygen atom bonded to $Y_{21}$ is bonded to a phosphorus atom of a phosphate ester of a terminal nucleotide in the other region of the Y2a region and the Y1a region, which is not bonded to $Y_{11}$.

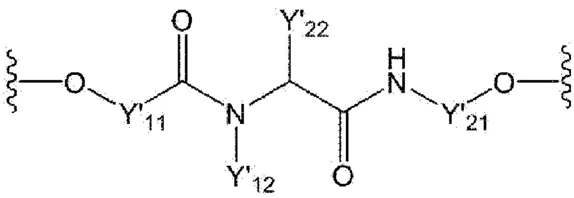

(I')

In the formula, $Y'_{11}$ and $Y'_{21}$ each independently represent an alkylene group having 1 to 20 carbon atoms, and $Y'_{12}$ and $Y'_{22}$ each independently represent a hydrogen atom or an alkyl group which may be substituted with an amino group, or $Y'_{12}$ and $Y'_{22}$ are bonded to each other at their terminals to represent an alkylene group having 3 or 4 carbon atoms,

a terminal oxygen atom bonded to $Y'_{11}$ is bonded to a phosphorus atom of a phosphate ester of a terminal nucleotide in any one region of the W1a region and the W2a region, and

a terminal oxygen atom bonded to $Y'_{21}$ is bonded to a phosphorus atom of a phosphate ester of a terminal nucleotide in the other region of the W2a region and the W1a region, which is not bonded to $Y'_{11}$.

[12] The production method according to [10] or [11], in which the divalent group represented by Formula (I) is a divalent group represented by Formula (II-A) or (II-B).

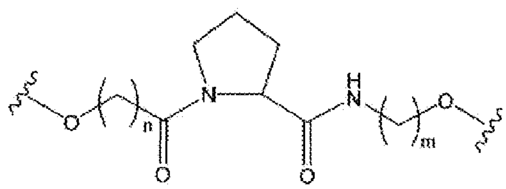

(II-A)

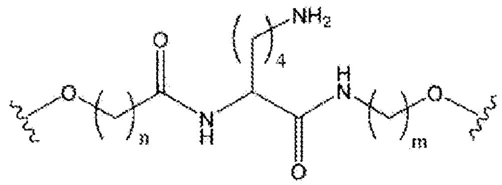

(II-B)

In the formulae, n and m each independently represent any integer of 1 to 20.

[13] The production method according to [11] or [12], in which the divalent group represented by Formula (I') is a divalent group represented by Formula (II-A) or (II-B).

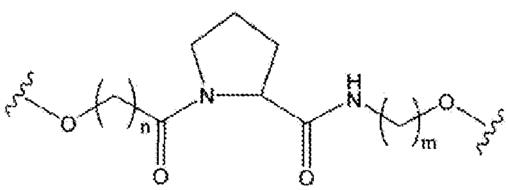

(II-A)

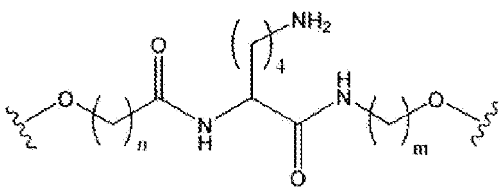

(II-B)

In the formulae, n and m each independently represent any integer of 1 to 20.

[14] The production method according to any one of [9] to [13], in which a nucleotide sequence that suppresses expression of a gene is included in at least one of a region consisting of the Y1a region, the X1a region, and the W1a region, and a region consisting of the X2a region and the Y2a region.

[15] The production method according to any one of [1] to [14], in which the RNA ligase is T4 bacteriophage-derived T4 RNA ligase 2, KVP40-derived ligase 2, Trypanosoma brucei RNA ligase, Deinococcus radiodurans RNA ligase,

or Leishmania tarentolae RNA ligase.

[16] The production method according to any one of [1] to [15], in which the RNA ligase is an RNA ligase consisting of an amino acid sequence having an identity of 95% or more with an amino acid sequence set forth in SEQ ID NO: 21, 22, or 23.

[17] The production method according to any one of [1] to [15], in which the RNA ligase is T4 bacteriophage-derived T4 RNA ligase 2 or KVP40-derived RNA ligase 2.

[18] The production method according to any one of [1] to [17], in which the ligase is T4 bacteriophage-derived T4 RNA ligase 2.

[Advantageous Effects of Invention]

According to the method of the present invention, a simple production method for a single-strand RNA is provided.

[Brief Description of Drawings]

Fig. 1 is a schematic diagram showing an example of first to third single-strand RNAs according to one embodiment of the present invention.

Fig. 2 is a schematic diagram showing another example of the first to third single-strand RNAs according to one embodiment of the present invention.

Fig. 3 is a diagram showing sequences used in Examples 1 and 2.

Fig. 4 is a diagram showing a linked single-strand RNA synthesized in Example 1.

Fig. 5 is a diagram showing results of liquid chromatography in Example 1.

Fig. 6 is a diagram showing a linked single-strand RNA synthesized in Example 2.

Fig. 7 is a diagram showing results of liquid chromatography in Example 2.

Fig. 8 is a schematic diagram showing an example of first and second single-strand RNAs according to one embodiment of the present invention.

Fig. 9 is a schematic diagram showing another example of the first and second single-strand RNAs according to one embodiment of the present invention.

[Description of Embodiments]

Hereinafter, the present invention will be described in detail.

As used herein, "comprise" includes the meaning of "essentially consist of" and the meaning of "consist of'.

As used herein, a "gene" includes a double-strand DNA, a single-strand DNA (a sense strand or an antisense strand), and fragments thereof, unless otherwise specified. Moreover, the "gene" in the present invention is used to indicate a regulatory region, a coding region, an exon, and an intron without distinction, unless otherwise specified.

As used herein, a numerical range of the number of nucleotides indicates, for example, all positive integers belonging to the range, and as a specific example, a description of "one to four nucleotides" means indication of all of "one nucleotide", "two nucleotides", "three nucleotides", and "four nucleotides". Moreover, a description of "one to four bases" means indication of all of "one base", "two bases", "three bases", and "four bases". As used herein "the number of nucleotides" and "the number of bases" interchangeably represent the length of a nucleotide sequence.

[First aspect]

A production method according to a first aspect of the present invention is a method for producing a single-strand RNA, including a step of causing an RNA ligase which is classified as EC 6.5.1.3 defined by the International Union of Biochemistry and Molecular Biology as an enzyme number and has a nick repair activity to act on a first single-strand RNA and a second single-strand RNA, which have a phosphate group at the 5' end, and a third single-strand RNA having a hydroxyl group at the 5' end to link the first single-strand RNA, the second single-strand RNA, and the third single-strand RNA,

in which a) the first single-strand RNA is a single-strand RNA consisting of an X1 region, a W1 region, an Lw linker region, and a W2 region in order from the 5' end,

b) the second single-strand RNA is a single-strand RNA consisting of a Z2 region, an Lz linker region, a Z1 region, and a Y1 region in order from the 5' end,

c) the third single-strand RNA is a single-strand RNA consisting of an X2 region and a Y2 region in order from the 5' end,

d) the X1 region and the X2 region have nucleotide sequences which are complementary to each other and have the same number of nucleotides of two or more,

e) the Y1 region and the Y2 region have nucleotide sequences which are complementary to each other and have the same number of nucleotides of two or more,

f) the Z1 region and the Z2 region have nucleotide sequences which are complementary to each other and have the same number of nucleotides of one or more,

g) the W1 region and the W2 region each independently have any number of nucleotide sequences,

h) the Lz linker region and the Lw linker region are each independently a linker region having an atomic group derived from an amino acid, and

i) a single-strand RNA generated by linking the first single-strand RNA, the second single-strand RNA, and the third single-strand RNA is a linked single-strand RNA consisting of the X2 region, the Y2 region, the Z2 region, the Lz linker region, the Z1 region, the Y1 region, the X1 region, the W1 region, the Lw linker region, and the W2 region in order from the 5' end.

This aspect can be implemented in a form in which a single-strand RNA which is generated from the first single-strand RNA and the second RNA strand, and consists of the Z2 region, the Lz linker region, the Z1 region, the Y1 region, the X1 region, the W1 region, the Lw linker region, and the W2 region in order from the 5' end is reacted with the third RNA, or a form in which a single-strand RNA which is generated from the second single-strand RNA and the third RNA strand, and consists of the X2 region, the Y2 region, the Z2 region, the Lz linker region, the Z1 region, and the Y1 region in order from the 5' end is reacted with the first RNA.

Regarding the linked single-strand RNA generated by the production method according to the present aspect, reference can be made to PCT International Publication No. WO2012/005368, PCT International Publication No. WO2012/017919, PCT International Publication No. WO2013/103146, and the like.

The number of bases (excluding the Lw linker region) in the first single-strand RNA used in the production method according to the present aspect is not particularly limited, the lower limit thereof is, for example, 4 and preferably 8, and the upper limit thereof is, for example, 160 and preferably 30. The range of the number of bases in the first single-strand RNA is, for example, 4 to 160 and preferably 8 to 30.

The number of bases (excluding the Lz linker region) in the second single-strand RNA used in the production method according to the present aspect is not particularly limited, the lower limit thereof is, for example, 6 and preferably 8, and the upper limit thereof is, for example, 150 and preferably 30. The range of the number of bases in the second single-strand RNA is, for example, 6 to 150 and preferably 8 to 30.

The number of bases in the third single-strand RNA used in the production method according to the present aspect is not particularly limited, the lower limit thereof is, for example, 4 and preferably 8, and the upper limit thereof is, for example, 160 and preferably 30. The range of the number of bases in the third single-strand RNA is, for example, 4 to 160 and preferably 8 to 30.

The X1 region and the X2 region have nucleotide sequences which are complementary to each other. The lower limit of the number of bases in the complementary nucleotide sequences is 2 and preferably 4, and the upper limit thereof is, for example, 150 and preferably 24. The range of the number of bases in the complementary nucleotide sequences is, for example, 2 to 150 and preferably 4 to 24.

The Y1 region and the Y2 region have nucleotide sequences which are complementary to each other. The lower limit of the number of bases in the complementary nucleotide sequences is 2 and preferably 4, and the upper limit thereof is, for example, 100 and preferably 30. The range of the number of bases in the complementary nucleotide sequences is, for example, 2 to 100 and preferably 4 to 30.

The Z1 region and the Z2 region have nucleotide sequences which are complementary to each other. The lower limit of the number of bases in the complementary nucleotide sequences is 1, preferably 2, and more preferably 4, and the upper limit thereof is, for example, 100 and preferably 30. The range of the number of bases in the complementary nucleotide sequences is, for example, 1 to 100, preferably 2 to 30, and more preferably 4 to 30.

The number of bases in a nucleotide sequence included in each of the W1 region and the W2 region is not particularly limited, the lower limit thereof is, for example, 1 and preferably 4, and the upper limit thereof is, for example, 100 and preferably 10.

The range of the number of bases in the nucleotide sequence is, for example, 1 to 100 and preferably 4 to 10.

The number of bases (excluding the linker region) in the single-strand RNA produced by the production method according to the present aspect is not particularly limited, the lower limit thereof is, for example, 38 and preferably 42, and the upper limit thereof is, for example, 300 and preferably 80. The range of the number of bases in the single-strand RNA is, for example, 38 to 300 and preferably 42 to 80.

The single-strand RNA produced by the production method according to the present aspect desirably has a

nucleotide sequence suppressing expression of a gene, in at least one of a region (an A1 region) consisting of the Z1 region, the Y1 region, the X1 region, and the W1 region, and a region (a B1 region) consisting of the X2 region, the Y2 region, and the Z2 region. A method for suppressing the expression of the gene is not particularly limited, and an RNA interference method is preferable.

RNA interference (RNAi) refers to a phenomenon in which by introducing, into a cell, a double-strand RNA that consists of a sense RNA consisting of the same sequence as at least a part of an mRNA sequence of a target gene, and an antisense RNA consisting of a sequence complementary to the sense RNA, the mRNA of the target gene is degraded, and as a result, inhibition of translation into a protein is induced and expression of the target gene is inhibited. There are still some unknown details about the mechanism of the RNA interference, but it is considered that a main mechanism is that an enzyme (a type of the RNase III nucleolytic enzyme family) called DICER contacts a double-strand RNA, and the double-strand RNA is degraded into a small fragment called a siRNA.

A gene to be a target is not particularly limited, and a desired gene can be appropriately selected. A nucleotide sequence that suppresses expression of the gene to be a target is not particularly limited as long as the nucleotide sequence is a sequence capable of suppressing the gene expression, and can be designed by a conventional method based on sequence information of the target gene registered in a known database (for example, GenBank and the like) or the like. The nucleotide sequence has an identity of preferably 90% or more, more preferably 95% or more, still more preferably 98% or more, and most preferably 100% with a predetermined region of the gene to be a target.

As the nucleotide sequence that suppresses the expression of the target gene by RNA interference, for example, the sense RNA consisting of the same sequence as at least a part of the mRNA sequence of the target gene can be used. The number of bases in the nucleotide sequence that suppresses the expression of the target gene by RNA interference is not particularly limited, and is, for example, 19 to 30 and preferably 19 to 21.

Any one or both of the A1 region and the B1 region may have two or more identical expression suppression sequences for the same target gene, may have two or more different expression suppression sequences for the same target, or may have two or more different expression suppression sequences for different target genes. When the A1 region has two or more expression suppression sequences, an arrangement position of each expression suppression sequence is not particularly limited, and may be any one or more regions of the X1 region, the Y1 region, and the Z1 region or may be a region spanning two or more regions. When the B1 region has two or more expression suppression sequences, an arrangement position of each expression suppression sequence may be any one or more regions of the X2 region, the Y2 region, and the Z2 region or may be a region spanning two or more regions. The expression suppression sequence has a complementarity of preferably 90% or more, more preferably 95%, still more preferably 98%, and particularly preferably 100% with respect to a predetermined region of the target gene.

Such a linked single-strand RNA is produced through a step of causing a ligase to act on the first single-strand RNA and the second single-strand RNA, which have a phosphate group at the 5' end, and the third single-strand RNA having a hydroxyl group at the 5' end to link the first single-strand RNA, the second single strand, and the third single strand (see Fig. 1).

In the linked single-strand RNA, sequence portions having a complementarity are arranged in a molecule and a double strand can be partially formed in the molecule. As shown in Fig. 1, in a linked single-strand RNA molecule, the X1 region and the X2 region have nucleotide sequences complementary to each other, the Y1 region and the Y2 region have nucleotide sequences complementary to each other, the Z1 region and the Z2 region have nucleotide sequences complementary to each other, a double strand is formed between these sequences having a complementarity, and the Lw and Lz linker regions take loop structures according to a length thereof. Fig. 1 merely shows the linking order of the regions and a positional relationship between the respective regions forming a double-strand portion, and for example, the length of each region, the shape of the linker region (Lw and Lz), and the like are not limited thereto.

At least one of the A1 region consisting of the Z1 region, the Y1 region, the X1 region, and the W1 region, and the B region consisting of the X2 region and the Y2 region may have at least one sequence that suppresses the expression of the target gene by the RNA interference. For example, the A1 region may have the sense RNA sequence consisting of the same sequence as at least a part of the mRNA sequence of the target gene, and the B1 region may have the antisense RNA sequence complementary to the sense RNA. Alternatively, for example, the B1 region may have the sense RNA sequence consisting of the same sequence as at least a part of the mRNA sequence of the target gene, and the A1 region may have the antisense RNA sequence complementary to the sense RNA.

The single-strand RNA produced by the production method according to the present aspect may consist of a ribonucleotide, may contain a deoxynucleotide, a non-nucleotide, and the like, or may contain a phosphorothioester bond in which some of oxygen atoms of a phosphorodiester bond in a ribonucleotide are substituted with sulfur atoms. The single-strand RNA produced by the production method according to the present aspect preferably consists of a ribonucleotide.

The Lz linker region and the Lw linker region are each independently a linker region having an atomic group derived from an amino acid, and the amino acid here may be any one of a natural amino acid and an artificial amino acid and may have a substituent or a protective group.

Examples of the Lz linker region include a divalent group represented by Formula (1).

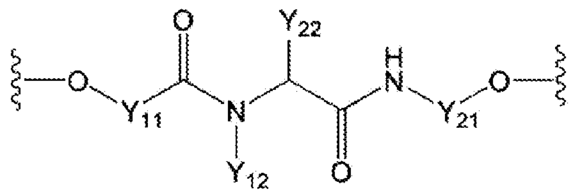

(I)

In the formula, $Y_{11}$ and $Y_{21}$ each independently represent an alkylene group having 1 to 20 carbon atoms, and $Y_{12}$ and $Y_{22}$ each independently represent a hydrogen atom or an alkyl group which may be substituted with an amino group, or $Y_{12}$ and $Y_{22}$ are bonded to each other at their terminals to represent an alkylene group having 3 or 4 carbon atoms,

a terminal oxygen atom bonded to $Y_{11}$ is bonded to a phosphorus atom of a phosphate ester of a terminal nucleotide in any one of the Z1 region and the Z2 region, and

a terminal oxygen atom bonded to $Y_{21}$ is bonded to a phosphorus atom of a phosphate ester of a terminal nucleotide in the other of the Z2 region and the Z1 region, which is not bonded to $Y_{11}$.

In addition, examples of the Lw linker region include a divalent group represented by Formula (I').

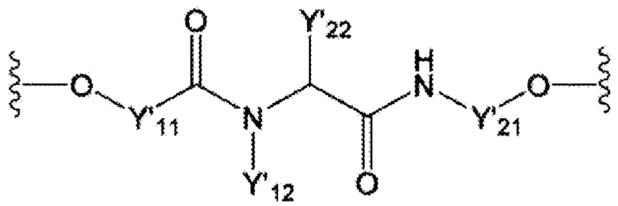

(I')

In the formula, $Y'_{11}$ and $Y'_{21}$ each independently represent an alkylene group having 1 to 20 carbon atoms, and $Y'_{12}$ and $Y'_{22}$ each independently represent a hydrogen atom or an alkyl group which may be substituted with an amino group, or $Y'_{12}$ and $Y'_{22}$ are bonded to each other at their terminals to represent an alkylene group having 3 or 4 carbon atoms,

a terminal oxygen atom bonded to $Y'_{11}$ of the linker Lw region is bonded to a phosphorus atom of a phosphate ester of a terminal nucleotide in any one of the W1 region and the W2 region, and

a terminal oxygen atom bonded to $Y'_{21}$ is bonded to a phosphorus atom of a phosphate ester of a terminal nucleotide in the other of the W2 region and the W1 region, which is not bonded to $Y'_{11}$.

The alkylene group having 1 to 20 carbon atoms may be linear or branched, and is preferably an alkylene group having 4 to 6 carbon atoms. Examples of the alkylene group include a methylene group, an ethylene group, an n-propylene group, an isopropylene group, an n-butylene group, an isobutylene group, a tert-butylene group, an n-pentylene group, an isopentylene group, and a hexylene group.

The alkyl group in the alkyl group which may be substituted with an amino group may be linear or branched, and is preferably an alkyl group having 1 to 10 carbon atoms and more preferably an alkyl group having 1 to 5 carbon atoms. Examples of the alkyl group include a methyl group, an ethyl group, an n-propyl group, an isopropyl group, an n-butyl group, an isobutyl group, a tert-butyl group, an n-pentyl group, an isopentyl group, and a hexyl group. As the alkyl group which may be substituted with an amino group, a group in which some of hydrogen atoms of the exemplified alkyl group are substituted with amino groups is also an exemplary example.

The number of amino groups that the alkyl group which may be substituted with the amino group can have is not particularly limited, and is preferably 1. Moreover, a substitution position of the amino group in the alkyl group which may be substituted with the amino group is also not particularly limited, and may be any position. Examples of the alkyl group which may be substituted with an amino group include a 4-aminobutyl group.

Preferred specific examples of the divalent group represented by any one of Formulae (I) and (I') include a divalent group represented by Formula (II-A) or (II-B).

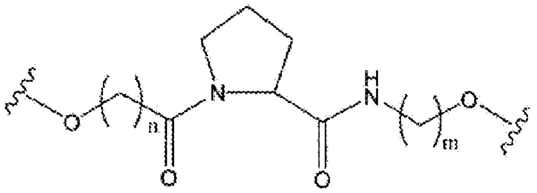

(II-A)

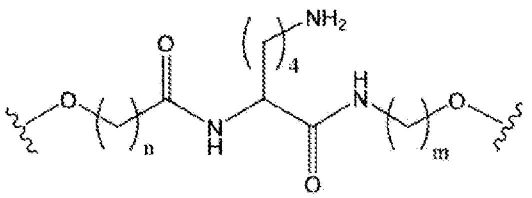

(II-B)

In the formulae, n and m each independently represent any integer of 1 to 20.

n and m are each preferably 4 to 6, and n and m may be the same as or different from each other.

An example of the first to third single-strand RNAs used in the production method according to the present aspect is shown in Figs. 1 and 2. The upper schematic diagram of Fig.1 shows the first to third single-strand RNAs, and the lower schematic diagram of Fig. 1 shows a state where a single-strand RNA is formed by bonding with a ligase. Fig. 2 shows a state where the first to third single-strand RNAs are linearly arranged.

In the sequences shown in the present specification and the drawings, the abbreviation of "p" indicates that a hydroxyl group at the 5' end is modified with a phosphate group. Moreover, the abbreviation of "P" in the sequences is a structure which is introduced by using an amidite represented by Structural Formula (III-A) and indicates a structure represented by the following formula.

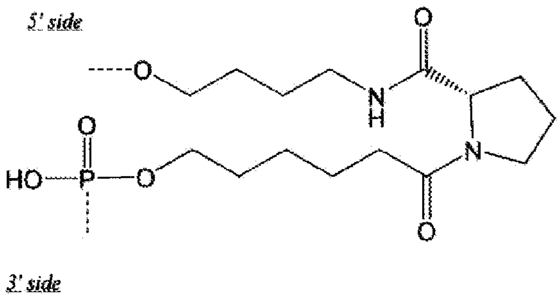


(III)-A

A method for producing the first to third single-strand RNAs used in the production method according to the present aspect is not particularly limited, the single-strand RNAs can be chemically synthesized by using a known solid-phase synthesis method or liquid-phase synthesis method, a known nucleic acid synthesizer, or the like. Examples of such a synthetic method include a phosphoramidite method, an H-phosphonate method, and the method described in PCT International Publication No. WO2013/027843.

For phosphorylation at the 5'-position of the single-strand RNA, an amidite for phosphorylation at the 5' end may be used in solid-phase synthesis. A commercially available amidite can be used as the amidite for 5' phosphorylation. Moreover, in the solid-phase synthesis, an RNA molecule having a hydroxyl group at the 5' end is synthesized, deprotection is performed, then phosphorylation is performed with a commercially available phosphorylating agent, and thus a single-strand RNA having a phosphate group at the 5' end can be prepared. As the phosphorylating agent, for example, a commercially available Chemical Phosphorylation Reagent (Glen Research) represented by Structural Formula (III-a) or the like is known (European Patent Application, Publication No. 0816368).

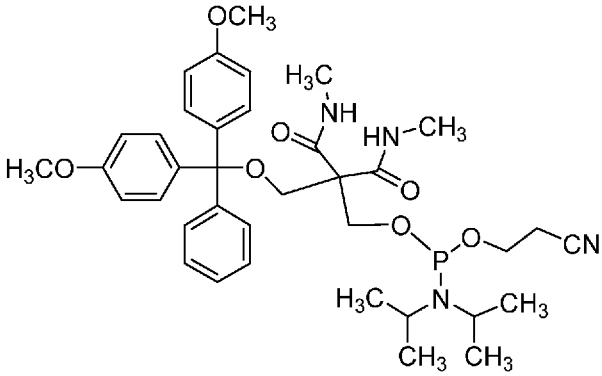

(III-a)

In addition, the Lz and Lw linker regions can be similarly prepared by a nucleic acid synthesizer using an amidite as shown below. As an amidite of the linker region, for example, an amidite having a proline skeleton, which is represented by Structural Formula (III-b), can be prepared by the method described in Example A4 in PCT International Publication No. WO2012/017919, and the following amidites represented by Structural Formulae (III-c), (III-d), and (III-e) can be prepared by the methods described in Examples A1 to A3 in PCT International Publication No. WO2013/103146.

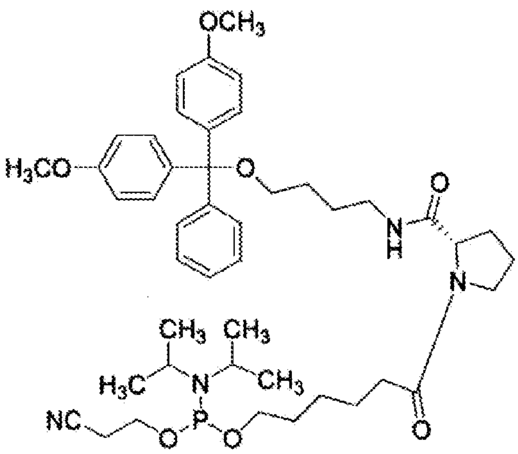

(III-b)

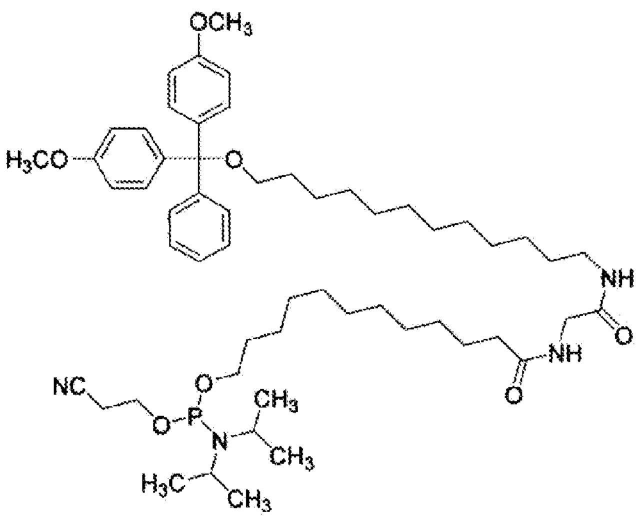

(III-c)

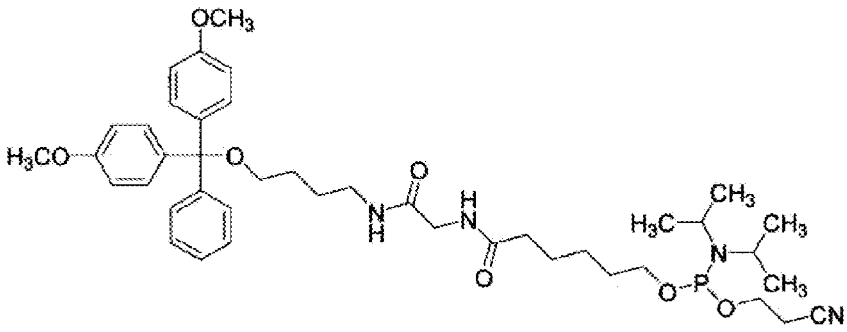

(III-d)

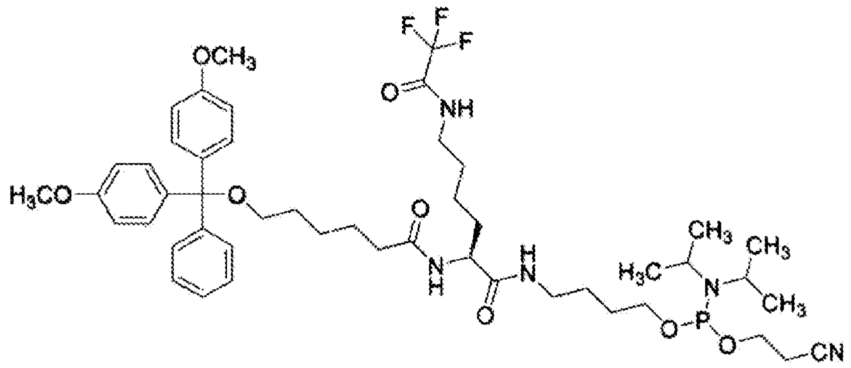

(III-e)

The RNA ligase used in the production method according to the present aspect is classified as EC 6.5.1.3 defined by the International Union of Biochemistry and Molecular Biology as an enzyme number, and has a nick repair activity. The RNA ligase is an enzyme which catalyzes a reaction of ATP + (ribonucleotide)$_n$-3'-hydroxyl + 5'-phospho-(ribonucleotide)$_m$ <=> (ribonucleotide)$_{n+m}$ + AMP + diphosphate, and has an activity of repairing a nick in a double-strand nucleic acid.

Examples of such an RNA ligase include T4 bacteriophage-derived T4 RNA ligase 2. The RNA ligase 2 can be purchased from, for example, (New England Biolabs). Furthermore, examples of the RNA ligase include vibriophage KVP40-derived ligase 2, Trypanosoma brucei RNA ligase, Deinococcus radiodurans RNA ligase, or Leishmania tarentolae RNA ligase. As such an RNA ligase, for example, a ligase obtained by performing extraction and purification from each organism by the method described in Non-Patent Document (Structure and Mechanism of RNA Ligase, Structure, Vol. 12, pp. 327 to 339) can also be used.

As the T4 bacteriophage-derived T4 RNA ligase 2, an RNA ligase which is a protein consisting of an amino acid sequence having an identity of 95% or more with an amino acid sequence set forth in SEQ ID NO: 10 and has a double strand nick repair activity can also be used. As such an RNA ligase 2, in addition to an enzyme having the amino acid sequence set forth in SEQ ID NO: 10, T39A, F65A, and F66A (see RNA ligase structures reveal the basis for RNA specificity and conformational changes that drive ligation forward, Cell. Vol. 127, pp.71 to 84), which are mutants thereof, can be exemplified. Such an RNA ligase 2 can be obtained, for example, by a method using Escherichia coli bacteriophage T4 which is deposited with the American Type Culture Collection (ATCC) as ATCC (registered trademark) 11303, based on the description in the above-mentioned literature, or a method such as PCR.

The KVP40-derived RNA ligase 2 can be obtained by the method described in Non-Patent Document (Characterization of bacteriophage KVP40 and T4 RNA ligase 2, Virology, vol. 319, pp. 141 to 151). Specifically, for example, the KVP40-derived RNA ligase 2 can be obtained by the following method. That is, in DNAs extracted from bacteriophage KVP40 (for example, deposited with JG1 as an accession number of Go008199), an open reading frame 293 is amplified by a polymerase chain reaction after restriction enzyme digestion with NdeI and BamHI. The obtained DNA is incorporated into a plasmid vector pET16b (Novagen). Alternatively, the DNA sequence can be artificially synthesized by PCR. Here, a desired mutant can be obtained by DNA sequence analysis. Subsequently, the obtained vector DNA is incorporated into E. coli BL21(DE3), and the resultant is cultured in an LB medium containing 0.1 mg/mL ampicillin. Isopropyl-β-thiogalactoside is added thereto so that a concentration is 0.5 mM, and cultured at 37°C for 3 hours. All subsequent operations are preferably performed at 4°C. First, bacterial cells are precipitated by a centrifugal operation, and the precipitate is stored at -80°C. A buffer A [50 mM Tris-HCl (pH of 7.5), 0.2 M NaCl, and 10% sucrose] is added to the frozen bacterial cells. Then, lysozyme and Triton X-100 are added thereto, and the bacterial cells are disrupted by ultrasonic waves to elute a target substance. Thereafter, the target substance is isolated by using affinity chromatography, size exclusion chromatography, or the like. Then, the obtained aqueous solution is subjected to centrifugal filtration, and

the resultant can be used as a ligase by replacing an eluate with a buffer.

In this way, the KVP40-derived RNA ligase 2 can be obtained. As the KVP40-derived RNA ligase 2, an RNA ligase which is a protein consisting of an amino acid sequence having an identity of 95% or more with an amino acid sequence of SEQ ID NO: 11 and has a double strand nick repair activity can be used.

The Deinococcus radiodurans RNA ligase can be obtained by the method described in Non-Patent Document (An RNA Ligase from Deinococcus radiodurans, J Biol Chem., Vol. 279, No. 49, pp. 50654 to 61). For example, the ligase can also be obtained from a biological material which is deposited with the ATCC as ATCC (registered trademark) BAA-816. As the Deinococcus radiodurans RNA ligase, an RNA ligase which is a protein consisting of an amino acid sequence having an identity of 95% or more with an amino acid sequence of SEQ ID NO: 12 and has a double strand nick repair activity can be used. Specific examples of such a ligase include an RNA ligase consisting of an amino acid sequence having a mutation of K165A or E278A in the RNA ligase of SEQ ID NO: 12, in addition to the RNA ligase consisting of the amino acid sequence of SEQ ID NO: 12 (An RNA Ligase from Deinococcus radiodurans, J Biol Chem., Vol. 279, No. 49, pp. 50654 to 61).

The Trypanosoma brucei RNA ligase can be obtained by the method described in Non-Patent Document (Assiciation of Two Novel Proteins TbMP52 and TbMP48 with the Trypanosoma brucei RNA Editing Complex, Vol. 21, No. 2, pp. 380 to 389).

The Leishmania tarentolae RNA ligase can be obtained by the method described in Non-Patent Document (The Mitochondrial RNA Ligase from Leishmania tarentolae Can Join RNA Molecules Bridged by a Complementary RNA, Vol. 274, No. 34, pp. 24289 to 24296).

Among them, as the RNA ligase, T4 bacteriophage-derived T4 RNA ligase 2 is preferable.

In the production method according to the present aspect, reaction conditions for causing the RNA ligase to act on the first to third single-strand RNAs are not particularly limited as long as the conditions are conditions under which the RNA ligase can function to produce a desired single-strand RNA, and can be appropriately set.

Regarding a used amount of the RNA ligase, a proper used amount can be appropriately set depending on amounts of the first to third single-strand RNAs to be linked, a reaction temperature, and a reaction time. The reaction time of the RNA ligase is selected from a range of 0.5 to 144 hours, for example. The pH in the RNA ligase treatment can be appropriately set according to the optimum pH of the used enzyme, and is selected from a pH range of 4 to 9, for example. The temperature in the RNA ligase treatment can be appropriately set according to the optimum temperature of the used enzyme, and is selected from a range of 0°C to 50°C, for example.

A reaction solution for causing the RNA ligase to act on the first to third single-strand RNAs may contain, in addition to these, a buffer solution, a metal salt, ATP, and the like. Examples of the buffer solution include a tris-hydrochloric acid buffer solution, an ATP buffer solution, a potassium phosphate buffer solution, a glycine-hydrochloric acid buffer solution, an acetate buffer solution, and a citrate buffer solution. Examples of the metal salt include a magnesium salt, a potassium salt, a calcium salt, and a sodium salt.

A crude product containing the single-strand RNA produced by causing the RNA ligase to act can usually be isolated by using a method for precipitating, extracting, and purifying the RNA. Specifically, a method for precipitating an RNA by adding a solvent having low solubility to the RNA, such as ethanol or isopropyl alcohol, to a solution after a reaction, or a method for adding a solution of phenol/chloroform/isoamyl alcohol (for example, phenol/chloroform/isoamyl alcohol = 25/24/1) to a reaction solution and extracting an RNA into an aqueous layer is employed. Thereafter, isolation and purification can be performed by a technique of known high-performance liquid chromatography (HPLC) such as reverse-phase column chromatography, anion-exchange column chromatography, and affinity column chromatography.

[Second aspect]

Next, a second aspect of the present invention will be described.

The second aspect of the present invention is a method for producing a single-strand RNA, including a step of causing an RNA ligase which is classified as EC 6.5.1.3 defined by the International Union of Biochemistry and Molecular Biology as an enzyme number and has a nick repair activity to act on a first single-strand RNA and a second single-strand RNA which have a phosphate group at the 5' end to link the first single-strand RNA and the second single-strand RNA. A single-strand RNA generated by linking the first single-strand RNA and the second single-strand RNA is a linked single-strand RNA consisting of an X2a region, a Y2a region, an Lz linker region, a Y1a region, an X1a region, a W1a region, an Lw linker region, and a W2a region in order from the 5' end (see Fig. 9). The production method according to the present aspect is defined by the following a) to g).

a) The first single-strand RNA is a single-strand RNA consisting of the Y2a region, the Lz linker region, the Y1a region, the X1a region, the W1a region, the Lw linker region, and the W2a region in order from the 5' end.
b) The second single-strand RNA is a single-strand RNA consisting of an X2a region.
c) The X1a region and the X2a region have nucleotide sequences which are complementary to each other and have

the same number of nucleotides of four or more.

d) The Y1a region and the Y2a region have nucleotide sequences which are complementary to each other and have the same number of nucleotides of one or more.

e) The W1a region and the W2a region each independently have any number of nucleotide sequences.

f) The Lw linker region and the Lz linker region are each independently a linker region having an atomic group derived from an amino acid. The Lw linker region and the Lz linker region are as described in the first aspect.

The linked single-strand RNA may have a nucleotide sequence suppressing expression of a gene, in at least one of an A region consisting of the Y1a region, the X1a region, and the W1a region, and a B region consisting of the X2a region and the Y2a region. As such a nucleotide sequence, a sequence that suppresses expression of a target gene by the RNA interference method may be included.

Any one or both of the A region and the B region may have two or more identical expression suppression sequences for the same target gene, may have two or more different expression suppression sequences for the same target, or may have two or more different expression suppression sequences for different target genes. When the A region has two or more expression suppression sequences, an arrangement position of each expression suppression sequence is not particularly limited, and may be any one or both of the X1a region and the Y1a region or may be a region spanning the both. When the B region has two or more expression suppression sequences, an arrangement position of each expression suppression sequence may be any one or both of the X2a region and the Y2a region or may be a region spanning the both. The expression suppression sequence has a complementarity of preferably 90% or more, more preferably 95%, still more preferably 98%, and particularly preferably 100% with respect to a predetermined region of the target gene.

Such a linked single-strand RNA is produced through a step of causing a ligase to act on the first single-strand RNA having a phosphate group at the 5' end and the second single-strand RNA having a hydroxyl group at the 3' end to link the first single-strand RNA and the second single strand (see Fig. 9).

In the linked single-strand RNA, sequence portions having a complementarity are arranged in a molecule and a double strand can be partially formed in the molecule. As shown in Fig. 9, in a linked single-strand RNA molecule, the X1 a region and the X2a region have nucleotide sequences complementary to each other, the Y1a region and the Y2a region have nucleotide sequences complementary to each other, a double strand is formed between these sequences having a complementarity, and the Lw and Lz linker regions take loop structures according to a length thereof. Fig. 9 merely shows the linking order of the regions and the positional relationship between the respective regions forming a double-strand portion, and for example, the length of each region, the shape of the linker region (Lw and Lz), and the like are not limited thereto.

At least one of the A region consisting of the Y1a region, the X1a region, and the W1a region, and the B region consisting of the X2a region and the Y2a region may have at least one sequence that suppresses the expression of the target gene by the RNA interference. In the linked single-strand RNA, the A region and the B region may be completely complementary or may be non-complementary in one or several nucleotides, but are preferably completely complementary. The one or several nucleotides are, for example, one to three nucleotides and preferably one or two nucleotides. The Y1a region has a nucleotide sequence complementary to the entire region of the Y2a region. The Y1a region and the Y2a region have nucleotide sequences which are completely complementary to each other, and are nucleotide sequences having the same number of nucleotides of one or more. The X1a region has a nucleotide sequence complementary to the entire region of the X2a region. The X1a region and the X2a region have nucleotide sequences which are completely complementary to each other, and are nucleotide sequences having the same number of nucleotides of two or more. In the production method according to the present embodiment, the W1a region and the W2a region are each a region having a nucleotide sequence including any number of nucleotides, are not an essential sequence, and may adopt an aspect in which the number of nucleotides is 0 or an aspect in which one or more nucleotides are included.

The length of each region is exemplified below, but is not limited thereto.

In the linked single-strand RNA molecule, a relationship among the number (W2an) of nucleotides in the W2a region, the number (X2an) of nucleotides in the X2a region, and the number (Y2an) of nucleotides in the Y2 region satisfies, for example, a condition of Expression (1). The relationship among the number (W1an) of nucleotides in the W1a region, the number (X1an) of nucleotides in the X1a region, and the number (Y1an) of nucleotides in the Y1a region satisfies, for example, a condition of Expression (2).

$$\mathrm{W2an} \leq \mathrm{X2an} + \mathrm{Y2an} \cdots (1)$$

$$\mathrm{W1an} \leq \mathrm{X1an} + \mathrm{Y1an} \cdots (2)$$

In the linked single-strand RNA molecule, the relationship between the number (X1an) of nucleotides in the X1 region and the number (Y1an) of nucleotides in the Y1a region is not particularly limited, and satisfies, for example, any one condition of the following expressions.

$$X1an = Y1an \cdots (3)$$

$$X1an < Y1an \cdots (4)$$

$$X1an > Y1an \cdots (5)$$

In the method according to the present embodiment, the number (X1an) of nucleotides in the X1a region, that is, the number (X2an) of nucleotides in the X2a region is 2 or more, preferably 4 or more, and more preferably 10 or more.

The number (Y1an) of nucleotides in the Y1a region, that is, the number (Y2an) of nucleotides in the Y2a region is 1 or more, preferably 2 or more, and more preferably 4 or more.

The W1a region preferably has a nucleotide sequence complementary to the entire region of the W2a region or a partial region of the W2a region. The W1a region and the W2a region may be non-complementary in one or several nucleotides, but are preferably completely complementary.

More specifically, the W2a region preferably consists of a nucleotide sequence shorter than that of the W1a region by one or more nucleotides. In this case, the entire nucleotide sequence of the W2a region is complementary to all nucleotides in any partial region of the W1a region. The nucleotide sequence from the 5' end to the 3' end of the W2a region is more preferably a sequence which is complementary to a nucleotide sequence starting from a nucleotide at the 3' end of the W1a region toward the 5' end.

In the linked single-strand RNA molecule, the relationship between the number (X1an) of nucleotides in the X1a region and the number (X2an) of bases in the X2a region, the relationship between the number (Y1an) of nucleotides in the Y1a region and the number (Y2an) of nucleotides in the Y2a region, and the relationship between the number (W1an) of nucleotides in the W1a region and the number (W2an) of nucleotides in the W2a region satisfy conditions of Expressions (6), (7), and (8), respectively.

$$X1an = X2an \cdots (6)$$

$$Y1an = Y2an \cdots (7)$$

$$W1an \geq W2an \cdots (8)$$

In the linked single-strand RNA, the lower limit of the sum of the number of nucleotides excluding the linker region (Lw and Lz) is typically 38, preferably 42, more preferably 50, still more preferably 51, and particularly preferably 52, and the upper limit thereof is typically 300, preferably 200, more preferably 150, still more preferably 100, and particularly preferably 80.

In the linked single-strand RNA, the lengths of the Lw and Lz linker regions are not particularly limited. For example, these linker regions preferably have a length such that the X1a region and the X2a region can form a double strand, or a length such that the Y1a region and the Y2a region can form a double strand. Each of the linker regions Lw and Lz is a region having an atomic group derived from an amino acid. These linker regions (Lw and Lz) are usually non-nucleotide linker regions.

The upper limit of the number of atoms forming a main chain of the linker region is typically 100, preferably 80, and more preferably 50.

The Lw linker region is, for example, a divalent group represented by Formula (I), and the Lz linker is, for example, a divalent group represented by Formula (I').

In the second aspect, reaction conditions for causing the RNA ligase to act on the first and second single-strand RNAs are not particularly limited as long as the conditions are conditions under which the RNA ligase can function to produce a desired single-strand RNA, and can be appropriately set.

As the RNA ligase, the same ligases as those exemplified in the first aspect can be used. Regarding a used

amount of the RNA ligase, a proper used amount can be appropriately set depending on amounts of the first to third single-strand RNAs to be linked, a reaction temperature, and a reaction time. The reaction time of the RNA ligase is selected from a range of 0.5 to 144 hours, for example. The pH in the RNA ligase treatment can be appropriately set according to the optimum pH of the used enzyme, and is selected from a pH range of 4 to 9, for example. The temperature in the RNA ligase treatment can be appropriately set according to the optimum temperature of the used enzyme, and is selected from a range of 0°C to 50°C, for example.

A reaction solution for causing the RNA ligase to act on the first and second single-strand RNAs may contain, in addition to these, a buffer solution, a metal salt, ATP, and the like. Examples of the buffer solution include a tris-hydrochloric acid buffer solution, an ATP buffer solution, a potassium phosphate buffer solution, a glycine-hydrochloric acid buffer solution, an acetate buffer solution, and a citrate buffer solution. Examples of the metal salt include a magnesium salt, a potassium salt, a calcium salt, and a sodium salt.

A crude product containing the single-strand RNA produced by causing the RNA ligase to act can usually be isolated by using a method for precipitating, extracting, and purifying the RNA. Specifically, a method for precipitating an RNA by adding a solvent having low solubility to the RNA, such as ethanol or isopropyl alcohol, to a solution after a reaction, or a method for adding a solution of phenol/chloroform/isoamyl alcohol (for example, phenol/chloroform/isoamyl alcohol = 25/24/1) to a reaction solution and extracting an RNA into an aqueous layer is employed. Thereafter, isolation and purification can be performed by a technique of known high-performance liquid chromatography (HPLC) such as reverse-phase column chromatography, anion-exchange column chromatography, and affinity column chromatography.

Among the single-strand RNAs produced by the method of the present invention, a single-strand RNA capable of suppressing expression of a gene to be a target can be used as an agent for treating or preventing diseases caused by the gene.

A case where a single-strand RNA having a length of about 20 bases is chemically synthesized is superior in terms of a yield rate and a yield, compared with a case where a single-strand RNA having a length of about 50 bases or more is chemically synthesized. For that reason, by chemically synthesizing the single-strand RNA having a length of about 20 bases instead of the chemical synthesis of the single-strand RNA having a length of about 50 bases or more to perform linkage due to ligation, a high yield rate and a high yield are obtained, RNA strands which have not been ligated can be easily removed by purification, and thus the chemical synthesis of the single-strand RNA having a length of about 20 bases is also excellent in terms of quality.

Therefore, according to the production method of the present invention, the single-strand RNA having a length of about 50 bases or more can be produced with a high yield rate, a high yield, and high quality. Moreover, by incorporating the nucleotide sequence that suppresses the expression of the target gene by the RNA interference, as a result, a single-strand RNA which can be used in the RNA interference method can be efficiently produced, and cost reduction is expected.

[Examples]

Hereinafter, Examples will be given to describe the present invention in more detail. However, the present invention is not limited to these Examples or the like.

· Example 1

1. Synthesis of first single-strand RNA

The following single-strand RNA (a strand I in Fig. 3) was synthesized. The strand has a length of 11 bases and corresponds to the first single-strand RNA.

Strand I: pCUCUGCUUCPG (5'-3')

The single-strand RNA was synthesized from a 3' side to a 5' side using a nucleic acid synthesizer (trade name NTS M-4MX-E, NIHON TECHNO SERVICE CO., LTD.) based on a phosphoramidite method.

The synthesis was performed by using the uridine EMM amidite described in Example 2, the cytidine EMM amidite described in Example 3, the adenosine EMM amidite described in Example 4, and the guanosine EMM amidite described in Example 5 in PCT International Publication No. WO2013/027843 and the proline amidite (IIIb) described in Example A3 in PCT International Publication No. WO2012/017919 as an RNA amidite, using the Chemical Phosphorylation Reagent (Glen Research) for 5' phosphorylation, using porous glass as a solid-phase carrier, using a high-purity trichloroacetic acid-toluene solution as a deblocking solution, using 5-benzylmercapto-1H-tetrazole as a condensing agent, using an iodine solution as an oxidizing agent, and using a phenoxyacetic acid solution and an N-methylimidazole solution as a capping solution.

Excision from the solid-phase carrier after solid-phase synthesis and deprotection followed the method described in PCT International Publication No. WO2013/027843. That is, after an ammonia aqueous solution and ethanol were added and the mixture was allowed to stand for a while, the solid-phase carrier was filtered and then deprotection of a hydroxyl group was performed using tetrabutylammonium fluoride. The obtained RNA was dissolved in distilled water for injection so as to have a desired concentration.

2. Synthesis of second single-strand RNA

The following single-strand RNA (a strand II in Fig. 1) was synthesized. The strand has a length of 26 bases and corresponds to the second single-strand RNA.

Strand II: pCAUAUACCPGGUAUAUGCUGUGUGUA (5'-3') (SEQ ID NO: 1)

The single-strand RNA was synthesized by the same method as above.

3. Synthesis of third single-strand RNA

The following single-strand RNA (a strand III in Fig. 1) was synthesized. The strand has a length of 16 bases and corresponds to the third single-strand RNA.

Strand III: AGCAGAGUACACACAG (5'-3') (SEQ ID NO: 2)

The single-strand RNA was synthesized by the same method as above.

4. Ligation

Next, a ligation reaction was performed at a reaction scale of 0.2 mL with a composition of a 16.2 μM RNA double strand, 50 units/μL T4 RNA ligase 2, 50 mM Tris-HCl (pH of 7.5), 2 mM $MgCl_2$, 1 mM DTT, and 400 μM ATP.

A linked single-strand RNA obtained by ligating the first to third RNAs is shown below and in Fig. 4.

5'-

AGCAGAGUACACACAGCAUAUACCPGGUAUAUGCUGUGUGUACUCUGCUU

CPG-3' (SEQ ID NOs: 3 and 4)

Specifically, first, 128 μL of a buffer solution (10× buffer solution) of 500 mM Tris-HCl (pH of 7.5), 20 mM $MgCl_2$, 10 mM DTT, and 4000 μM ATP, and distilled water for injection was added into a 1.5-mL tube so that a concentration of the first RNA strand was 5.8 μM, a concentration of the second RNA strand was 5.8 μM, and a concentration of the third RNA strand was 7.6 μM, and the mixture was allowed to stand in a 37°C water bath for 10 minutes. Thereafter, the T4 RNA ligase 2 (New England Biolabs) was added so that the above composition and the above reaction scale were satisfied, and the mixture was incubated at 37°C for 3 hours. After the reaction, 50 μL of a reaction solution was added to 5 μL of 0.2 M ethylenediaminetetraacetic acid, and the solution was analyzed under LC conditions in Table 1.

[Table 1]

| | | | |
|---|---|---|---|
| Detector | UV 260 nm | | |
| Column | ACQU1TY UPLC Oligonucleotide BEH C18 manufactured by Waters Corporation, φ 2.1 × 100 mm, 1.7 μm | | |
| Column temperature | 80°C | | |
| Injected sample amount | 5 μL | | |
| Flow rate | 0.2 mL/min | | |
| Mobile phase A | 16 mM trimethylamine, 400 mM aqueous hexafluoro-2-propanol | | |
| Mobile phase B | Methanol/mobile phase A = 1/1 | | |
| Gradient conditions | Time (min) | 0 | 40 |
| | Concentration of mobile phase B | 15 | 45 |

(continued)

| | |
|---|---|
| Analysis time | 40 min |

As a result of the HPLC analysis, after the reaction, the first single-strand RNA, the second single-strand RNA, and the third single-strand RNA were decreased, and two new peaks with a delayed retention time were observed (Fig. 5).
As a result of checking the molecular weight of the elution peak by mass spectrometry measurement, it could be confirmed that a peak with a short retention time was a fourth single-strand RNA obtained by bonding the first single-strand RNA and the second single-strand RNA. Moreover, it could be confirmed that a peak with a long retention time was a target linked single-strand RNA obtained by bonding the first single-strand RNA, the second single-strand RNA, and the third single-strand RNA (measured value: 17025.4836 and theoretical value: 17025.4672).
The solution after the reaction was separated into respective components by HPLC (a wavelength of 260 nm), and the respective components were quantified from area values of the raw material and the product in the obtained chromatogram.
As a result of calculating a conversion rate from these quantitative values by the following expression, the conversion rate was 22%.

Conversion rate = {amount of linked single-strand RNA/(amount of second single-strand RNA + amount of fourth single-strand RNA + amount of linked single-strand RNA)}

· Example 2

1. Synthesis of first single-strand RNA

The following single-strand RNA (a strand IV in Fig. 3) was synthesized. The strand has a length of 14 bases and corresponds to the first single-strand RNA.
Strand IV: pGUACUCUGCUUCPG (5'-3') (SEQ ID NO: 5)
The single-strand RNA was synthesized by the same method as above.

2. Synthesis of second single-strand RNA

The following single-strand RNA (a strand V in Fig. 3) was synthesized. The strand has a length of 19 bases and corresponds to the second single-strand RNA.
Strand V: pUACCPGGUAUAUGCUGUGU (5'-3') (SEQ ID NO: 6)

3. Synthesis of third single-strand RNA

The following single-strand RNA (a strand VI in Fig. 3) was synthesized. The strand has a length of 20 bases and corresponds to the third single-strand RNA.
Strand VI: AGCAGAGUACACACAGCAUA (5'-3') (SEQ ID NO: 7)

4. Ligation

A linked single-strand RNA obtained by ligating the first to third RNAs is shown below and in Fig. 6.

5'-AGCAGAGUACACACAGCAUAUACCPGGUAUAUGCUGUGUGUACUCUGCUUCPG-3' (SEQ ID NOs: 3 and 4)

The ligation and the analysis were performed in the same manner as above.

As a result of the HPLC analysis, after the reaction, the first single-strand RNA, the second single-strand RNA, and the third single-strand RNA were decreased, and two new peaks with a delayed retention time were observed.

As a result of checking a molecular weight of the elution peak by the mass spectrometry measurement, it could be confirmed that a peak with a short retention time was a fourth single-strand nucleic acid obtained by bonding the second single-strand RNA and the third single-strand nuclear RNA. Moreover, it could be confirmed that a peak with a long retention time was a target linked single-strand RNA obtained by bonding the first single-strand RNA, the second single-strand RNA, and the third single-strand RNA (measured value: 17025.4779 and theoretical value: 17025.4672).

The solution after the reaction was separated into respective components by HPLC (a wavelength of 260 nm), and the respective components were quantified from area values of the raw material and the product in the obtained chromatogram.

As a result of calculating a conversion rate from these quantitative values by the following expression, the conversion rate was 71%.

$$\text{Conversion rate} = \{\text{amount of linked single-strand RNA}/(\text{amount of second single-strand RNA} + \text{amount of fourth single-strand RNA} + \text{amount of linked single-strand RNA})\}$$

· Example 3

1. Synthesis of single-strand RNA generated from first single-strand RNA and second single-strand RNA

The following single-strand RNA (a strand VII in Fig. 3) was synthesized. The strand has a length of 37 bases and corresponds to the RNA generated from the first single-strand RNA and the second single-strand RNA.

Strand VII: pCAUAUACCPGGUAUAUGCUGUGUGUACUCUGCUUCPG (5'-3') (SEQ ID NO: 4)

The single-strand RNA was synthesized by the same method as above.

2. Ligation

A linked single-strand RNA obtained by ligating the RNA generated from the first single-strand RNA and the second single-strand RNA to the third single-strand RNA (the strand III in Fig. 3) is shown below and in Fig. 6.

5'-CAUAUACCPGGUAUAUGCUGUGUGUACUCUGCUUCPG-3' (SEQ ID NOs: 3 and 4)

Next, a ligation reaction was performed at a reaction scale of 21 mL with a composition of a 16.2 μM RNA double strand, 50 units/μL T4 RNA ligase 2, 50 mM Tris-HCl (pH of 7.0), 2 mM $MgCl_2$, 1 mM DTT, and 400 μM ATP.

After the reaction, a reaction solution was purified by column chromatography under conditions shown in Table 2. The obtained fractions were analyzed by HPLC, and fractions (fractions in which a proportion occupied by a peak area of the target substance with respect to a total area of peaks detected in a chromatogram was 90% or more) having a purity of 90% or more were collected and mixed. As a result, a target substance could be obtained with a purity of 91 % and a total yield rate of 11 %. Furthermore, as a result of measuring the obtained sample by the mass spectrometry measurement, the measured value matched a calculated value, and thus it could be confirmed that the target substance was obtained (measured value: 17025.4752 and theoretical value: 17025.4672). Here, the total yield rate is a proportion of an amount of a single-strand RNA obtained by purifying a product after a ligation reaction with respect to an amount of a solid-phase carrier charged. The amount of the RNA is determined based on an absorbance of ultraviolet rays having a wavelength of 260 nm. The amount is a value obtained by assuming that a concentration of the RNA when the absorbance is 1 is 33 μg/mL.

[Table 2]

| | |
|---|---|
| Detector | UV 260 nm |
| Column | Triart C18 manufactured by YMC CO., LTD., 150 mm × 4.6 mm ID |
| Column temperature | 60°C |
| Flow rate | 1.0 mL/min |

(continued)

| | | | | | |
|---|---|---|---|---|---|
| Mobile phase A | 100 mM dibutylammonium acetate (pH of 7.0) | | | | |
| Mobile phase B | Acetonitrile | | | | |
| Fractionation width | 1.25 mL | | | | |
| Gradient conditions | Time (min) | 0 | 5 | 45 | 50 |
| | Concentration (%) of mobile phase B | 10 | 35 | 43 | 100 |

· Example 4

1. Synthesis of single-strand RNA generated from first single-strand RNA and second single-strand RNA

The following single-strand RNA (a strand VIII in Fig. 3) was synthesized. The strand has a length of 33 bases and corresponds to the RNA generated from the first single-strand RNA and the second single-strand RNA.

Strand VIII: pUACCPGGUAUAUGCUGUGUGUACUCUGCUUCPG (5'-3') (SEQ ID NO: 4)

The single-strand RNA was synthesized by the same method as above.

2. Ligation

A linked single-strand RNA obtained by ligating the RNA generated from the first single-strand RNA and the second single-strand RNA to the third RNA (the strand VI in Fig. 3) is shown below and in Fig. 6.

5'-

AGCAGAGUACACACAGCAUAUACCPGGUAUAUGCUGUGUGUACUCUGCUU

CPG-3' (SEQ ID NOs: 3 and 4)

Next, a ligation reaction was performed at a reaction scale of 19 mL with a composition of a 16.2 μM RNA double strand, 50 units/μL T4 RNA ligase 2, 50 mM Tris-HCl (pH of 7.0), 2 mM $MgCl_2$, 1 mM DTT, and 400 μM ATP.

After the reaction, a reaction solution was purified by column chromatography under conditions shown in Table 2. The obtained fractions were analyzed by HPLC, and fractions (fractions in which a proportion occupied by a peak area of the target substance with respect to a total area of peaks detected in a chromatogram was 90% or more) having a purity of 90% or more were collected and mixed. As a result, a target substance could be obtained with a purity of 94% and a total yield rate of 11%. Furthermore, as a result of measuring the obtained sample by the mass spectrometry measurement, the measured value matched a calculated value, and thus it could be confirmed that the target substance was obtained (measured value: 17025.4831 and theoretical value: 17025.4672). Here, the total yield rate is a proportion of an amount of a single-strand RNA obtained by purifying a product after a ligation reaction with respect to an amount of a solid-phase carrier charged. The amount of the RNA is determined based on an absorbance of ultraviolet rays having a wavelength of 260 nm. The amount is a value obtained by assuming that a concentration of the RNA when the absorbance is 1 is 33 μg/mL.

· Example 5

1. Synthesis of single-strand nucleic acid generated from first single-strand nucleic acid and second single-strand nucleic acid

The following single-strand nucleic acid (a strand IX in Fig. 3) was synthesized. The strand has a length of 31 bases and corresponds to the RNA generated from the first single-strand RNA and the second single-strand RNA.

Strand IX: pCCPGGUAUAUGCUGUGUGUACUCUGCUUCPG (5'-3') (SEQ ID NO: 4)

The single-strand RNA was synthesized by the same method as above.

2. Synthesis of third single-strand RNA

The following single-strand RNA (a strand X in Fig. 3) was synthesized. The strand has a length of 22 bases and corresponds to the second single-strand nucleic acid.
Strand X: AGCAGAGUACACACAGCAUAUA (5'-3') (SEQ ID NO: 8)

3. Ligation

A linked single-strand RNA obtained by ligating the first to third RNAs is shown below and in Fig. 6.

5'-

AGCAGAGUACACACAGCAUAUACCPGGUAUAUGCUGUGUGUACUCUGCUU

CPG-3' (SEQ ID NOs: 3 and 4)

Next, a ligation reaction was performed at a reaction scale of 17 mL with a composition of a 16.2 μM RNA double strand, 50 units/μL T4 RNA ligase 2, 50 mM Tris-HCl (pH of 7.0), 2 mM $MgCl_2$, 1 mM DTT, and 400 μM ATP.
After the reaction, a reaction solution was purified by column chromatography under conditions shown in Table 2. The obtained fractions were analyzed by HPLC, and fractions (fractions in which a proportion occupied by a peak area of the target substance with respect to a total area of peaks detected in a chromatogram was 90% or more) having a purity of 90% or more were collected and mixed. As a result, a target substance could be obtained with a purity of 93% and a total yield rate of 12%. Furthermore, as a result of measuring the obtained sample by the mass spectrometry measurement, the measured value matched a calculated value, and thus it could be confirmed that the target substance was obtained (measured value: 17025.5004 and theoretical value: 17025.4672). Here, the total yield rate is a proportion of an amount of a single-strand RNA obtained by purifying a product after a ligation reaction with respect to an amount of a solid-phase carrier charged. The amount of the RNA is determined based on an absorbance of ultraviolet rays having a wavelength of 260 nm. The amount is a value obtained by assuming that a concentration of the RNA when the absorbance is 1 is 33 μg/mL.

· Example 6

1. Synthesis of single-strand RNA generated from first single-strand RNA and second single-strand RNA

The following single-strand RNA (a strand XI in Fig. 3) was synthesized. The strand has a length of 30 bases and corresponds to the RNA generated from the first single-strand RNA and the second single-strand RNA.
Strand XI: pCPGGUAUAUGCUGUGUGUACUCUGCUUCPG (5'-3') (SEQ ID NO: 4)
The single-strand RNA was synthesized by the same method as above.

2. Synthesis of third single-strand RNA

The following single-strand RNA (a strand XII in Fig. 3) was synthesized. The strand has a length of 23 bases and corresponds to the second single-strand RNA.
Strand XII: AGCAGAGUACACACAGCAUAUAC (5'-3') (SEQ ID NO: 9)

3. Ligation

A linked single-strand RNA obtained by ligating the first to third RNAs is shown below and in Fig. 6.

5'-

AGCAGAGUACACACAGCAUAUACCPGGUAUAUGCUGUGUGUACUCUGCUU

CPG-3' (SEQ ID NOs: 3 and 4)

Next, a ligation reaction was performed at a reaction scale of 18 mL with a composition of a 16.2 μM RNA double strand, 50 units/μL T4 RNA ligase 2, 50 mM Tris-HCl (pH of 7.0), 2 mM $MgCl_2$, 1 mM DTT, and 400 μM ATP.

After the reaction, a reaction solution was purified by column chromatography under conditions shown in Table 2. The obtained fractions were analyzed by HPLC, and fractions (fractions in which a proportion occupied by a peak area of the target substance with respect to a total area of peaks detected in a chromatogram was 90% or more) having a purity of 90% or more were collected and mixed. As a result, a target substance could be obtained with a purity of 93% and a total yield rate of 17%. Furthermore, as a result of measuring the obtained sample by the mass spectrometry measurement, the measured value matched a calculated value, and thus it could be confirmed that the target substance was obtained (measured value: 17025.5031 and theoretical value: 17025.4672). Here, the total yield rate is a proportion of an amount of a single-strand RNA obtained by purifying a product after a ligation reaction with respect to an amount of a solid-phase carrier charged. The amount of the RNA is determined based on an absorbance of ultraviolet rays having a wavelength of 260 nm. The amount is a value obtained by assuming that a concentration of the RNA when the absorbance is 1 is 33 μg/mL.

## Claims

1. A method for producing a single-strand RNA, comprising:

    a step of causing an RNA ligase which is classified as EC 6.5.1.3 defined by the International Union of Biochemistry and Molecular Biology as an enzyme number and has a nick repair activity to act on a first single-strand RNA and a second single-strand RNA, which have a phosphate group at the 5' end, and a third single-strand RNA having a hydroxyl group at the 5' end to link the first single-strand RNA, the second single-strand RNA, and the third single-strand RNA,
    wherein a) the first single-strand RNA is a single-strand RNA consisting of an X1 region, a W1 region, an Lw linker region, and a W2 region in order from the 5' end,
    b) the second single-strand RNA is a single-strand RNA consisting of a Z2 region, an Lz linker region, a Z1 region, and a Y1 region in order from the 5' end,
    c) the third single-strand RNA is a single-strand RNA consisting of an X2 region and a Y2 region in order from the 5' end,
    d) the X1 region and the X2 region have nucleotide sequences which are complementary to each other and have the same number of nucleotides of two or more,
    e) the Y1 region and the Y2 region have nucleotide sequences which are complementary to each other and have the same number of nucleotides of two or more,
    f) the Z1 region and the Z2 region have nucleotide sequences which are complementary to each other and have the same number of nucleotides of one or more,
    g) the W1 region and the W2 region each independently have any number of nucleotide sequences,
    h) the Lz linker region and the Lw linker region are each independently a linker region having an atomic group derived from an amino acid, and
    i) a single-strand RNA generated by linking the first single-strand RNA, the second single-strand RNA, and the third single-strand RNA is a linked single-strand RNA consisting of the X2 region, the Y2 region, the Z2 region, the Lz linker region, the Z1 region, the Y1 region, the X1 region, the W1 region, the Lw linker region, and the W2 region in order from the 5' end.

2. The production method according to claim 1,
    wherein the Lz linker region is a divalent group represented by Formula (I), and

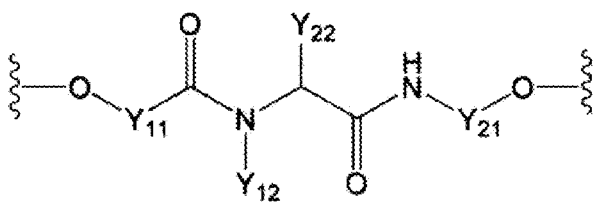 (I)

wherein in the formula, $Y_{11}$ and $Y_{21}$ each independently represent an alkylene group having 1 to 20 carbon atoms, and $Y_{12}$ and $Y_{22}$ each independently represent a hydrogen atom or an alkyl group which may be substituted with an amino group, or $Y_{12}$ and $Y_{22}$ are bonded to each other at their terminals to represent an alkylene group having 3 or 4 carbon atoms,
a terminal oxygen atom bonded to $Y_{11}$ is bonded to a phosphorus atom of a phosphate ester of a terminal nucleotide in any one of the Z1 region and the Z2 region, and
a terminal oxygen atom bonded to $Y_{21}$ is bonded to a phosphorus atom of a phosphate ester of a terminal nucleotide in the other region of the Z2 region and the Z1 region, which is not bonded to $Y_{11}$.

**3.** The production method according to claim 2,
wherein the Lz linker region is a divalent group represented by Formula (I), and the Lw linker region is a divalent group represented by Formula (I'),

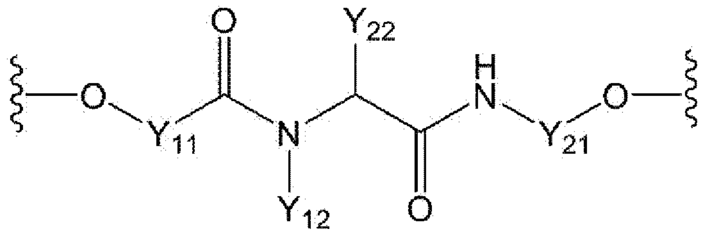
(I)

wherein in the formula, $Y_{11}$ and $Y_{21}$ each independently represent an alkylene group having 1 to 20 carbon atoms, and $Y_{12}$ and $Y_{22}$ each independently represent a hydrogen atom or an alkyl group which may be substituted with an amino group, or $Y_{12}$ and $Y_{22}$ are bonded to each other at their terminals to represent an alkylene group having 3 or 4 carbon atoms,
a terminal oxygen atom bonded to $Y_{11}$ is bonded to a phosphorus atom of a phosphate ester of a terminal nucleotide in any one region of the Z1 region and the Z2 region, and
a terminal oxygen atom bonded to $Y_{21}$ is bonded to a phosphorus atom of a phosphate ester of a terminal nucleotide in the other region of the Z2 region and the Z1 region, which is not bonded to $Y_{11}$, and

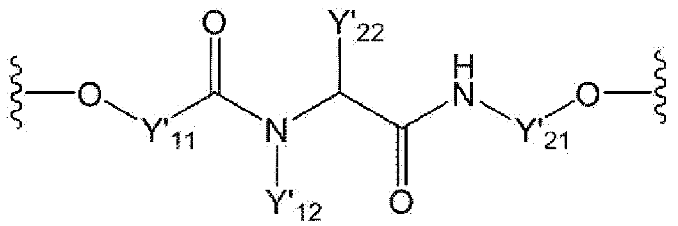
(I')

wherein in the formula, $Y'_{11}$ and $Y'_{21}$ each independently represent an alkylene group having 1 to 20 carbon atoms, and $Y'_{12}$ and $Y'_{22}$ each independently represent a hydrogen atom or an alkyl group which may be substituted with an amino group, or $Y'_{12}$ and $Y'_{22}$ are bonded to each other at their terminals to represent an alkylene group having 3 or 4 carbon atoms,
a terminal oxygen atom bonded to $Y'_{11}$ is bonded to a phosphorus atom of a phosphate ester of a terminal nucleotide in any one region of the W1 region and the W2 region, and
a terminal oxygen atom bonded to $Y'_{21}$ is bonded to a phosphorus atom of a phosphate ester of a terminal nucleotide in the other region of the W2 region and the W1 region, which is not bonded to $Y'_{11}$.

**4.** The production method according to claim 2 or 3,
wherein the divalent group represented by Formula (I) is a divalent group represented by Formula (II-A) or (II-B), and

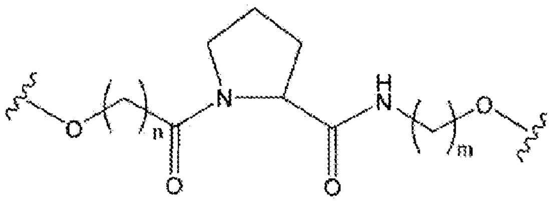

(II-A)

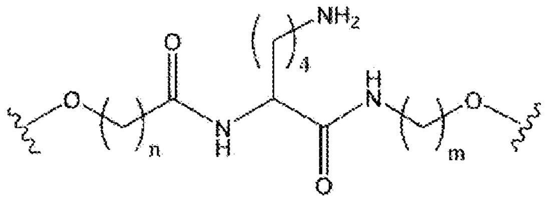

(II-B)

wherein in the formulae, n and m each independently represent any integer of 1 to 20.

**5.** The production method according to claim 3 or 4,
wherein the divalent group represented by Formula (I') is a divalent group represented by Formula (II-A) or (II-B), and

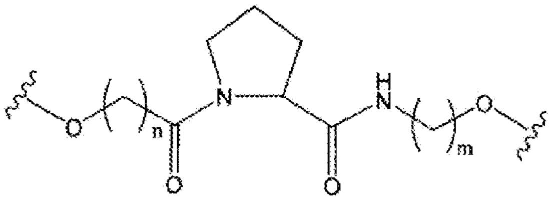

(II-A)

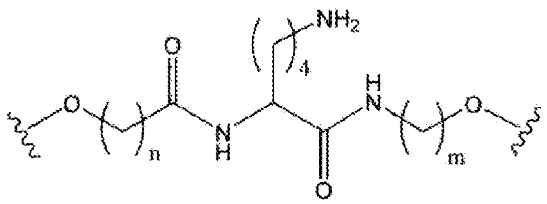

(II-B)

wherein in the formulae, n and m each independently represent any integer of 1 to 20.

**6.** The production method according to any one of claims 1 to 5,
wherein a single-strand RNA generated by linking the first single-strand RNA, the second single-strand RNA, and the third single-strand RNA has a nucleotide sequence suppressing expression of a gene, in at least one of a region consisting of the Z1 region, the Y1 region, the X1 region, and the W1 region, and a region consisting of the X2 region, the Y2 region, and the Z2 region.

**7.** The production method according to any one of claims 1 to 6,
wherein a single-strand RNA which is generated from the first single-strand RNA and the second RNA strand, and consists of the Z2 region, the Lz linker region, the Z1 region, the Y1 region, the X1 region, the W1 region, the Lw linker region, and the W2 region in order from the 5' end is reacted with the third RNA.

**8.** The production method according to any one of claims 1 to 6,
wherein a single-strand RNA which is generated from the second single-strand RNA and the third RNA strand, and consists of the X2 region, the Y2 region, the Z2 region, the Lz linker region, the Z1 region, and the Y1 region in order from the 5' end is reacted with the first RNA.

**9.** A method for producing a single-strand RNA, comprising:

a step of causing an RNA ligase which is classified as EC 6.5.1.3 defined by the International Union of Biochemistry and Molecular Biology as an enzyme number and has a nick repair activity to act on a first single-strand RNA and a second single-strand RNA which have a phosphate group at the 5' end to link the first single-strand RNA and the second single-strand RNA,
wherein a) the first single-strand RNA is a single-strand RNA consisting of a Y2a region, an Lz linker region, a Y1a region, an X1a region, a W1a region, an Lw linker region, and a W2a region in order from the 5' end,
b) the second single-strand RNA is a single-strand RNA consisting of an X2a region,
c) the X1a region and the X2a region have nucleotide sequences which are complementary to each other and have the same number of nucleotides of four or more,
d) the Y1a region and the Y2a region have nucleotide sequences which are complementary to each other and have the same number of nucleotides of one or more,
e) the W1a region and the W2a region each independently have any number of nucleotide sequences,
f) the Lw linker region and the Lz linker region are each independently a linker region having an atomic group derived from an amino acid, and
g) a single-strand RNA generated by linking the first single-strand RNA and the second single-strand RNA is a linked single-strand RNA consisting of the X2a region, the Y2a region, the Lz linker region, the Y1a region, the X1a region, the W1a region, the Lw linker region, and the W2a region in order from the 5' end.

**10.** The production method according to claim 9,
wherein the Lz linker region is a divalent group represented by Formula (I), and

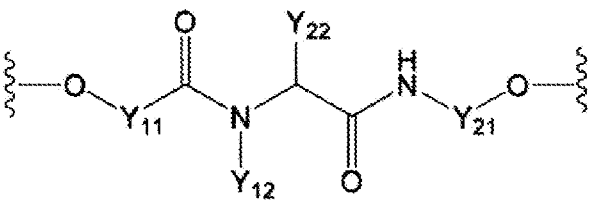

(I)

wherein in the formula, $Y_{11}$ and $Y_{21}$ each independently represent an alkylene group having 1 to 20 carbon atoms, and $Y_{12}$ and $Y_{22}$ each independently represent a hydrogen atom or an alkyl group which may be substituted with an amino group, or $Y_{12}$ and $Y_{22}$ are bonded to each other at their terminals to represent an alkylene group having 3 or 4 carbon atoms,
a terminal oxygen atom bonded to $Y_{11}$ is bonded to a phosphorus atom of a phosphate ester of a terminal nucleotide in any one of the Y1a region and the Y2a region, and
a terminal oxygen atom bonded to $Y_{21}$ is bonded to a phosphorus atom of a phosphate ester of a terminal nucleotide in the other of the Y1a region and the Y2a region, which is not bonded to $Y_{11}$.

**11.** The production method according to claim 9,
wherein the Lz linker region is a divalent group represented by Formula (I), and the Lw linker region is a divalent group represented by Formula (I'),

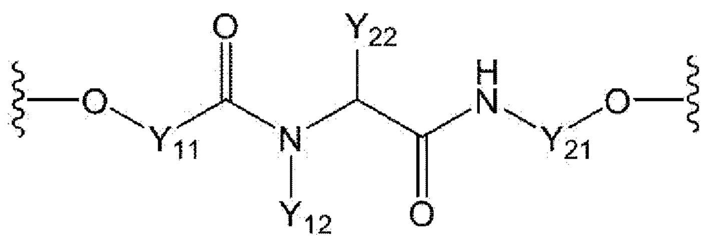

(I)

wherein in the formula, $Y_{11}$ and $Y_{21}$ each independently represent an alkylene group having 1 to 20 carbon atoms, and $Y_{12}$ and $Y_{22}$ each independently represent a hydrogen atom or an alkyl group which may be substituted with an amino group, or $Y_{12}$ and $Y_{22}$ are bonded to each other at their terminals to represent an alkylene group having 3 or 4 carbon atoms,
a terminal oxygen atom bonded to $Y_{11}$ is bonded to a phosphorus atom of a phosphate ester of a terminal nucleotide in any one region of the Y1a region and the Y2a region, and
a terminal oxygen atom bonded to $Y_{21}$ is bonded to a phosphorus atom of a phosphate ester of a terminal nucleotide in the other region of the Y2a region and the Y1a region, which is not bonded to $Y_{11}$, and

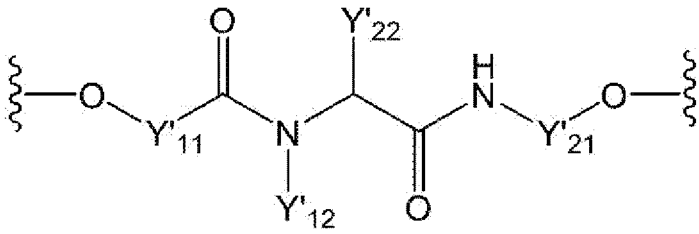

(I')

wherein in the formula, Y'$_{11}$ and Y'$_{21}$ each independently represent an alkylene group having 1 to 20 carbon atoms, and Y'$_{12}$ and Y'$_{22}$ each independently represent a hydrogen atom or an alkyl group which may be substituted with an amino group, or Y'$_{12}$ and Y'$_{22}$ are bonded to each other at their terminals to represent an alkylene group having 3 or 4 carbon atoms,
a terminal oxygen atom bonded to Y'$_{11}$ is bonded to a phosphorus atom of a phosphate ester of a terminal nucleotide in any one region of the W1a region and the W2a region, and
a terminal oxygen atom bonded to Y'$_{21}$ is bonded to a phosphorus atom of a phosphate ester of a terminal nucleotide in the other region of the W2a region and the W1a region, which is not bonded to Y'$_{11}$.

**12.** The production method according to claim 10 or 11,
wherein the divalent group represented by Formula (I) is a divalent group represented by Formula (II-A) or (II-B), and

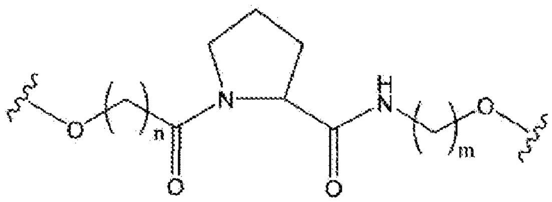

(II-A)

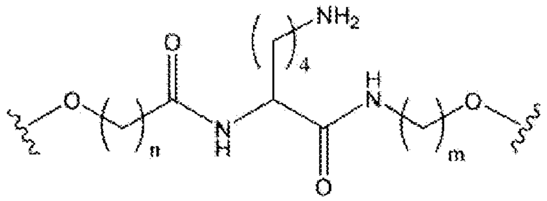

(II-B)

wherein in the formulae, n and m each independently represent any integer of 1 to 20.

**13.** The production method according to claim 11 or 12,
wherein the divalent group represented by Formula (I') is a divalent group represented by Formula (II-A) or (II-B), and

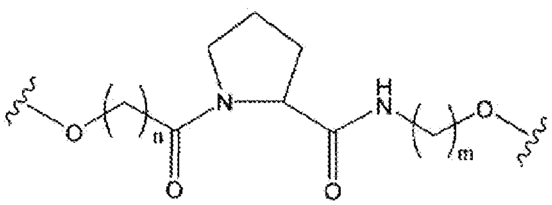

(II-A)

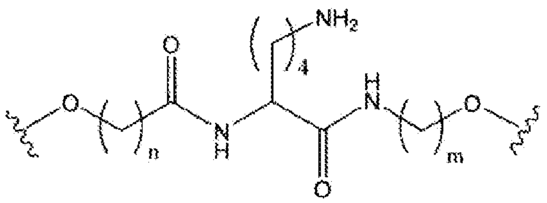

(II-B)

wherein in the formulae, n and m each independently represent any integer of 1 to 20.

**14.** The production method according to any one of claims 9 to 13,
wherein a single-strand RNA generated by linking the first single-strand RNA and the second single-strand RNA has a nucleotide sequence suppressing expression of a gene, in at least one of a region consisting of the Y1a region, the X1a region, and the W1a region, and a region consisting of the X2a region and the Y2a region.

**15.** The production method according to any one of claims 1 to 14,
wherein the RNA ligase is T4 bacteriophage-derived T4 RNA ligase 2, KVP40-derived ligase 2, Trypanosoma brucei RNA ligase, Deinococcus radiodurans RNA ligase, or Leishmania tarentolae RNA ligase.

**16.** The production method according to any one of claims 1 to 15,
wherein the RNA ligase is an RNA ligase consisting of an amino acid sequence having an identity of 95% or more with an amino acid sequence set forth in SEQ ID NO: 10, 11, or 12.

**17.** The production method according to any one of claims 1 to 15,
wherein the RNA ligase is T4 bacteriophage-derived T4 RNA ligase 2 or KVP40-derived RNA ligase 2.

**18.** The production method according to any one of claims 1 to 17,
wherein the ligase is T4 bacteriophage-derived T4 RNA ligase 2.

FIG. 1

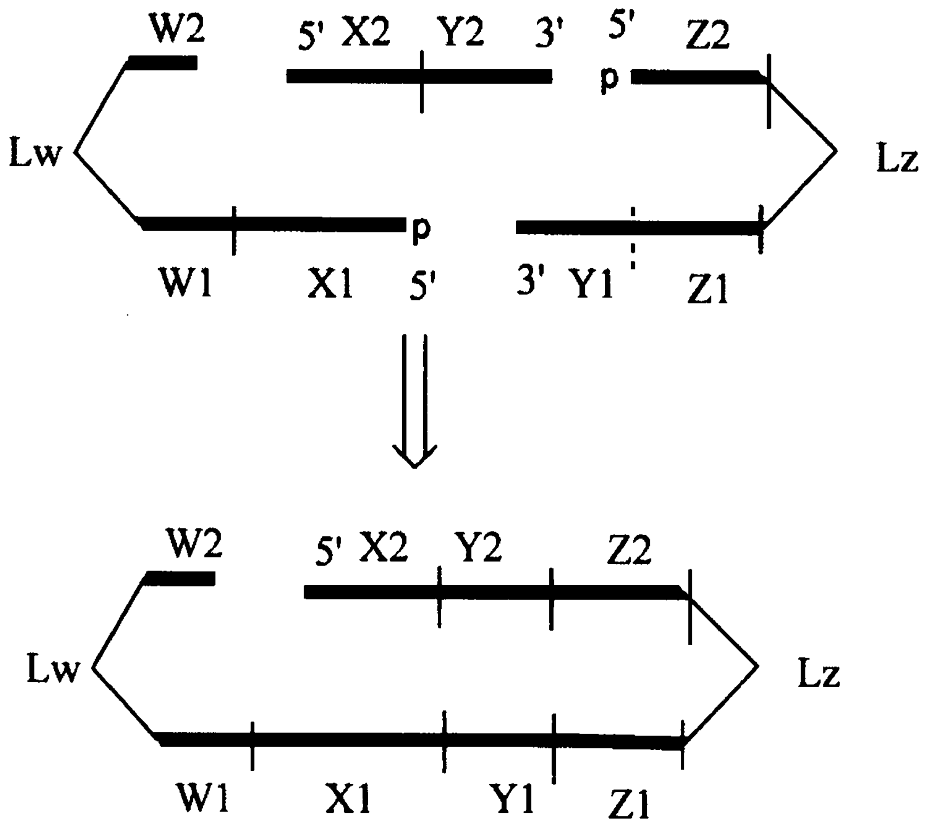
W2
5' X2
Y2
3'
5'
p
Z2
Lw
Lz
p
W1
X1
5'
3'
Y1
Z1
W2
5' X2
Y2
Z2
Lw
Lz
W1
X1
Y1
Z1

FIG. 2

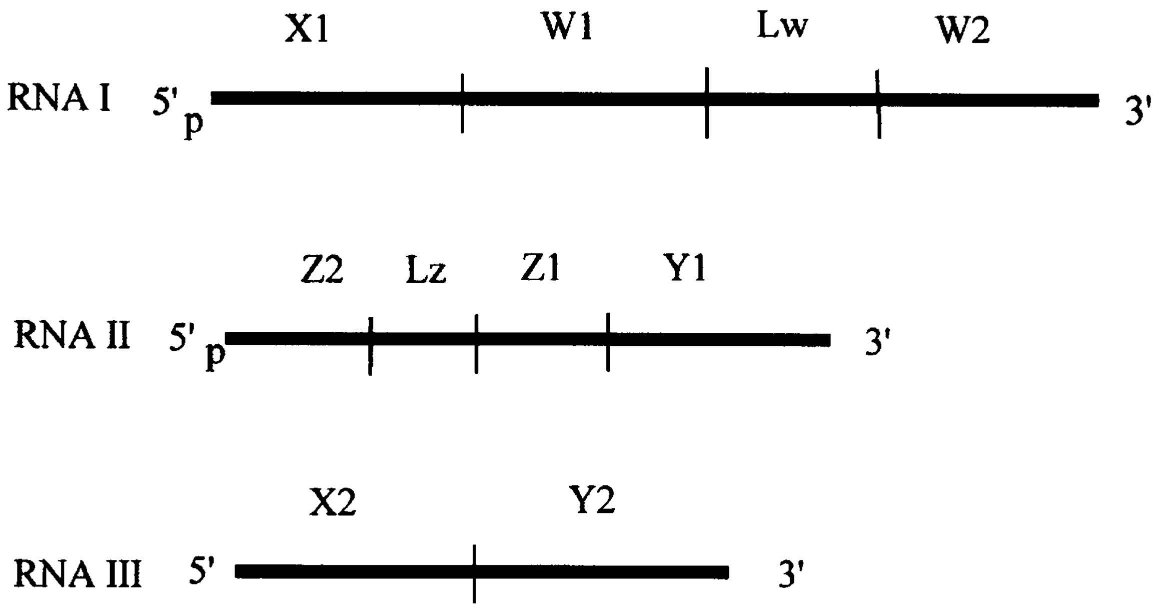
X1
W1
Lw
W2
RNA I
5'
p
3'
Z2
Lz
Z1
Y1
RNA II
5'
p
3'
X2
Y2
RNA III
5'
3'

# FIG. 3

| | |
|---|---|
| STRAND I | pCUCUGCUUCPG |
| STRAND II (SEQ ID NO: 1) | pCAUAUACCPGGUAUAUGCUGUGUGUA |
| STRAND III (SEQ ID NO: 2) | AGCAGAGUACACACAG |
| STRAND IV (SEQ ID NO: 5) | pGUACUCUGCUUCPG |
| STRAND V (SEQ ID NO: 6) | pUACCPGGUAUAUGCUGUGU |
| STRAND VI (SEQ ID NO: 7) | AGCAGAGUACACACAGCAUA |
| STRAND VII (SEQ ID NO: 4) | pCAUAUACCPGGUAUAUGCUGUGUGUACUCUGCUUCPG |
| STRAND VIII (SEQ ID NO: 4) | pUACCPGGUAUAUGCUGUGUGUACUCUGCUUCPG |
| STRAND IX (SEQ ID NO: 4) | pCCPGGUAUAUGCUGUGUGUACUCUGCUUCPG |
| STRAND X (SEQ ID NO: 8) | AGCAGAGUACACACAGCAUAUA |
| STRAND XI (SEQ ID NO: 4) | pCPGGUAUAUGCUGUGUGUACUCUGCUUCPG |
| STRAND XII (SEQ ID NO: 9) | AGCAGAGUACACACAGCAUAUAC |

# FIG. 4

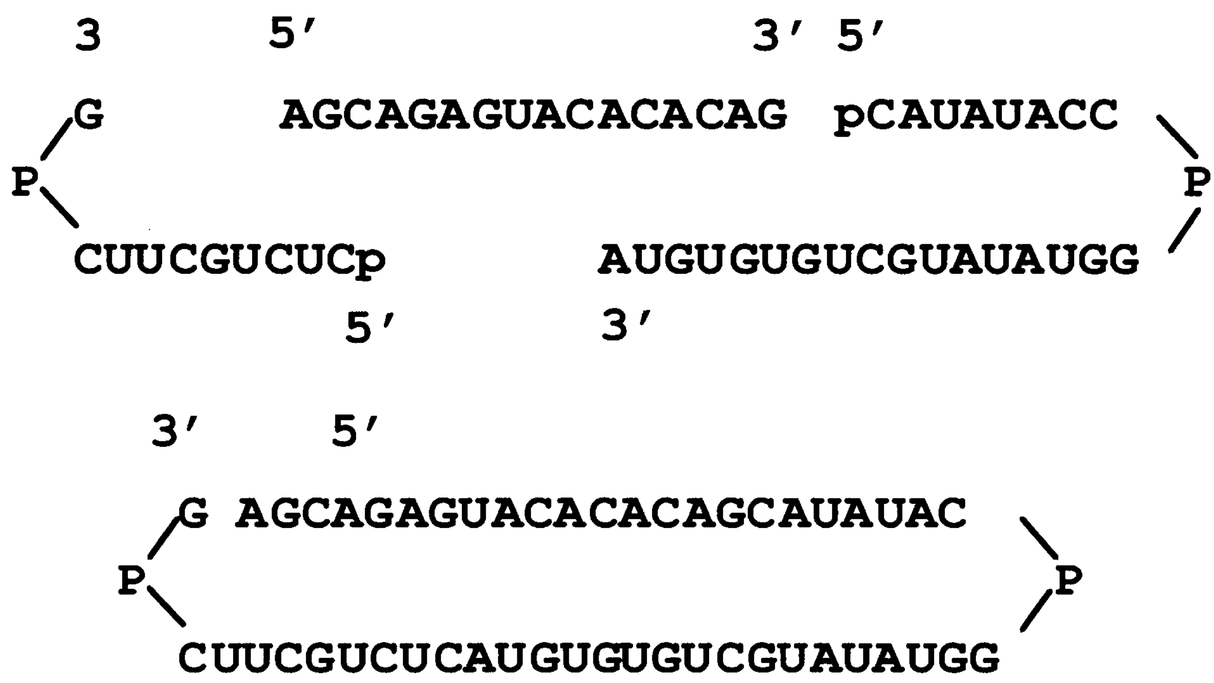

FIG. 5

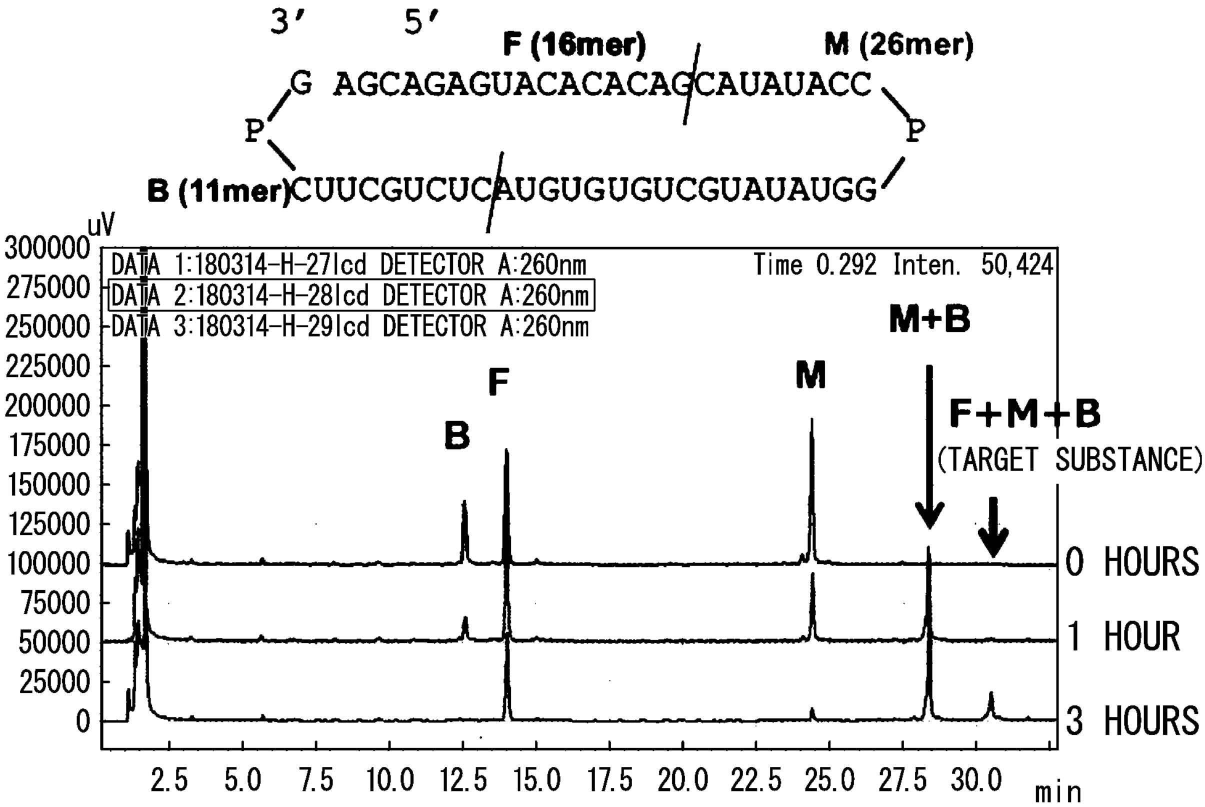
3′ 5′
F (16mer)
M (26mer)
G AGCAGAGUACACACAGCAUAUACC
P
P
B (11mer) CUUCGUCUCAUGUGUGUCGUAUAUGG
uV
DATA 1:180314-H-27lcd DETECTOR A:260nm
DATA 2:180314-H-28lcd DETECTOR A:260nm
DATA 3:180314-H-29lcd DETECTOR A:260nm
Time 0.292 Inten. 50,424
B
F
M
M+B
F+M+B
(TARGET SUBSTANCE)
0 HOURS
1 HOUR
3 HOURS
300000
275000
250000
225000
200000
175000
150000
125000
100000
75000
50000
25000
0
2.5 5.0 7.5 10.0 12.5 15.0 17.5 20.0 22.5 25.0 27.5 30.0
min

FIG. 6

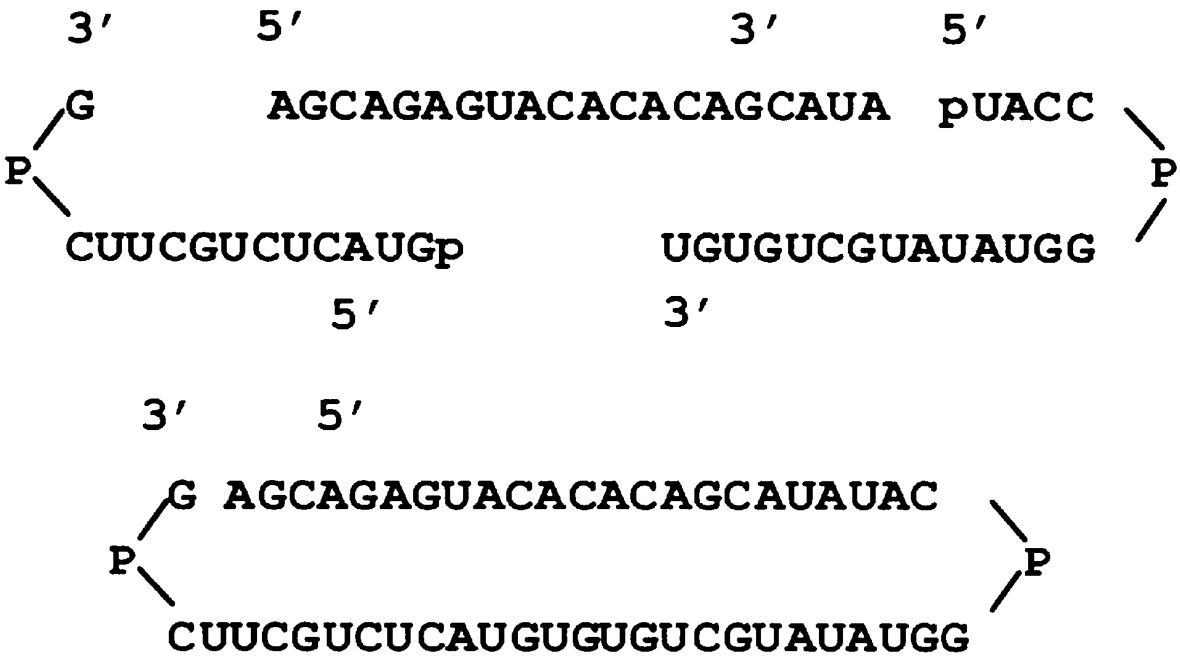
3′ 5′ 3′ 5′
G AGCAGAGUACACACAGCAUA pUACC
P
P
CUUCGUCUCAUGp UGUGUCGUAUAUGG
5′ 3′
3′ 5′
G AGCAGAGUACACACAGCAUAUAC
P
P
CUUCGUCUCAUGUGUGUCGUAUAUGG

FIG. 7

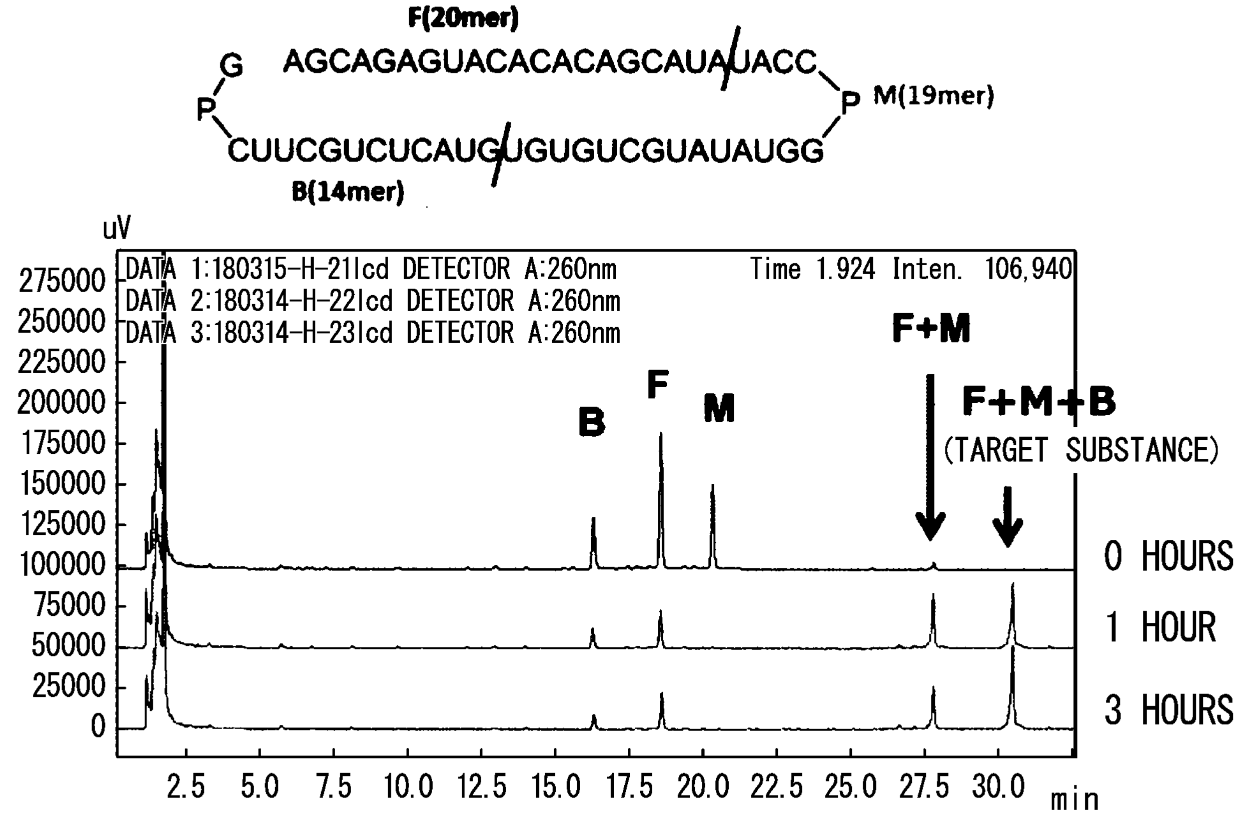
F(20mer)
G
AGCAGAGUACACACAGCAUAUACC
P
P M(19mer)
CUUCGUCUCAUGUGUGUCGUAUAUGG
B(14mer)
uV
DATA 1:180315-H-21lcd DETECTOR A:260nm
DATA 2:180314-H-22lcd DETECTOR A:260nm
DATA 3:180314-H-23lcd DETECTOR A:260nm
Time 1.924 Inten. 106,940
275000
250000
225000
200000
175000
150000
125000
100000
75000
50000
25000
0
B
F
M
F+M
F+M+B
(TARGET SUBSTANCE)
0 HOURS
1 HOUR
3 HOURS
2.5 5.0 7.5 10.0 12.5 15.0 17.5 20.0 22.5 25.0 27.5 30.0
min

FIG. 8

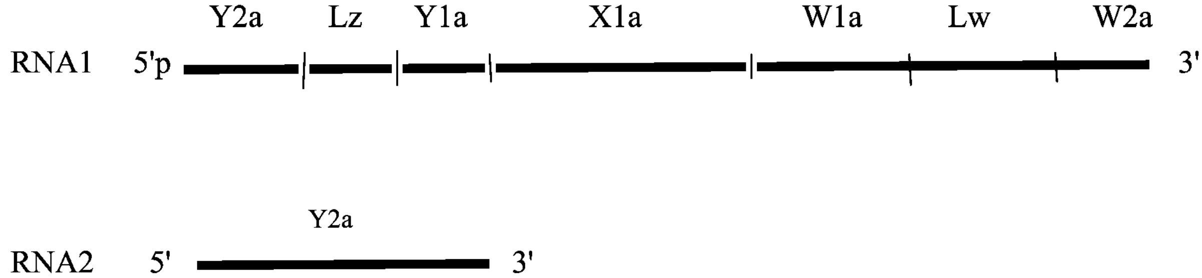
Y2a
Lz
Y1a
X1a
W1a
Lw
W2a
RNA1
5'p
3'
Y2a
RNA2
5'
3'

FIG. 9

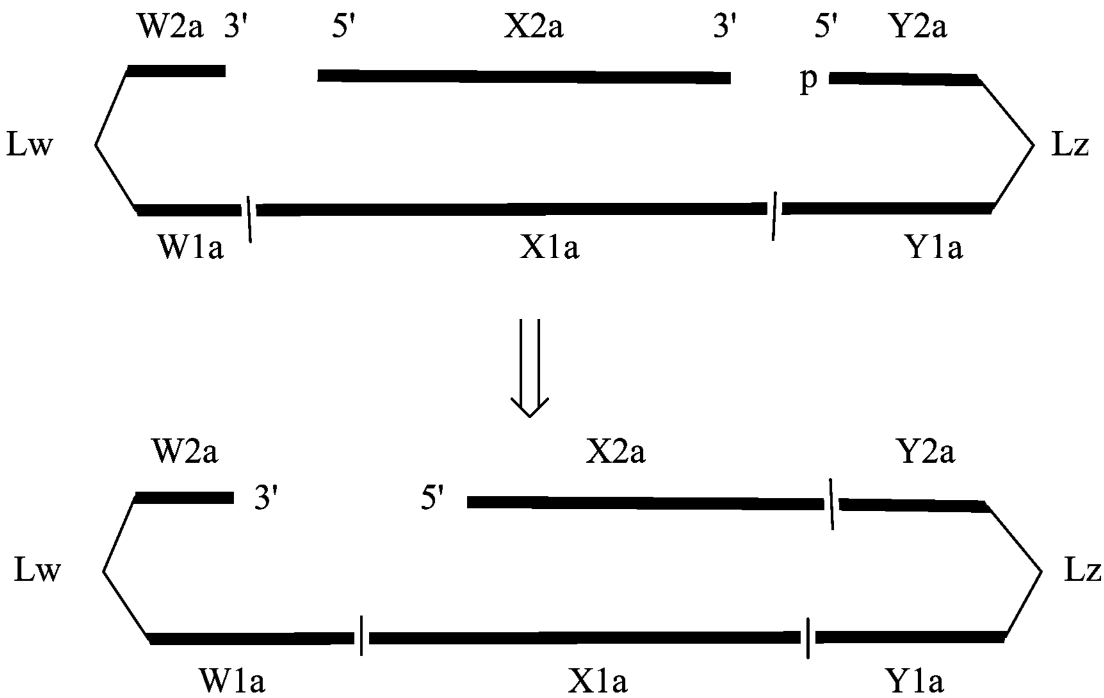
W2a
3'
5'
X2a
3'
5'
Y2a
p
Lw
Lz
W1a
X1a
Y1a
W2a
X2a
Y2a
3'
5'
Lw
Lz
W1a
X1a
Y1a

## INTERNATIONAL SEARCH REPORT

International application No.
PCT/JP2019/013631

A. CLASSIFICATION OF SUBJECT MATTER
Int.Cl. C12P19/34(2006.01)i, C12N15/10(2006.01)i, C12N15/113(2010.01)n

According to International Patent Classification (IPC) or to both national classification and IPC

B. FIELDS SEARCHED

Minimum documentation searched (classification system followed by classification symbols)
Int.Cl. C12P19/34, C12N15/00-15/90

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII), GenBank/EMBL/DDBJ/GeneSeq, PubMed, DWPI (Derwent Innovation)

C. DOCUMENTS CONSIDERED TO BE RELEVANT

| Category* | Citation of document, with indication, where appropriate, of the relevant passages | Relevant to claim No. |
|---|---|---|
| Y | WO 2013/103146 A1 (BONAC CORPORATION) 11 July 2013, claims 1-16, 32, paragraphs [0081]-[0103], [0110], [0156], [0218]-[0223], [0245]-[0247], [0262]-[0267], fig. 2-3, etc. & US 2014/0329886 A1, claims 1-16, 32, paragraphs [0101]-[0123], [0130], [0176], [0241]-[0246], [0276]-[0279], [0306]-[0310], fig. 2-3, etc. & EP 2801617 A1 & CN 104039961 A & KR 10-2014-0111688 A | 1-18 |
| Y | WO 2011/052013 A1 (BIODYNAMICS LABORATORY INC.) 05 May 2011, claim 1, paragraphs [0012], [0033], etc. (Family: none) | 1-18 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 June 2019 (14.06.2019) | 25 June 2019 (25.06.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/013631

C (Continuation). DOCUMENTS CONSIDERED TO BE RELEVANT

| Category* | Citation of document, with indication, where appropriate, of the relevant passages | Relevant to claim No. |
|---|---|---|
| A | WO 2012/017919 A1 (BONAC CORPORATION) 09 February 2012 & US 2012/0035246 A1 & EP 2436767 A1 & CN 103052711 A & KR 10-2013-0095738 A | 1-18 |
| A | WO 2012/005368 A1 (BONAC CORPORATION) 12 January 2012 & US 2012/0010271 A1 & EP 2431466 A1 & CN 102918156 A & KR 10-2013-0090792 A | 1-18 |
| A | JP 2016-502857 A (NEW ENGLAND BIOLABS, INC.) 01 February 2016 & US 2014/0179539 A1 & WO 2014/100473 A1 & EP 2935624 A1 & CN 105121655 A | 1-18 |
| A | JP 2003-505094 A (PHYLOS, INC.) 12 February 2003 & US 2002/0182687 A1 & WO 2001/007657 A1 & EP 1196637 A1 & KR 10-2002-0033743 A | 1-18 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018069560 A
- WO 2012017919 A
- WO 2012005368 A
- WO 2013103146 A
- WO 2013027843 A
- EP 0816368 A
- WO 2013027843 PCT


### Non-patent literature cited in the description


- Structure and Mechanism of RNA Ligase. *Structure,* vol. 12, 327-339
- RNA ligase structures reveal the basis for RNA specificity and conformational changes that drive ligation forward. *Cell,* vol. 127, 71-84
- Characterization of bacteriophage KVP40 and T4 RNA ligase 2. *Virology,* vol. 319, 141-151
- An RNA Ligase from Deinococcus radiodurans. *J Biol Chem.,* vol. 279 (49), 50654-61
- *Assiciation of Two Novel Proteins TbMP52 and TbMP48 with the Trypanosoma brucei RNA Editing Complex,* vol. 21 (2), 380-389
- *The Mitochondrial RNA Ligase from Leishmania tarentolae Can Join RNA Molecules Bridged by a Complementary RNA,* vol. 274 (34), 24289-24296

SEQUENCE LISTING

<110> Sumitomo Chemical Co., Ltd.

<120> Method for producing nucleic acid molecule

<130> S43021WO01

<150> JP2018-069560
<151> 2018-03-30

<160> 12

<170> PatentIn version 3.5

<210> 1
<211> 17
<212> RNA
<213> Artificial Sequence

<220>
<223> a part of single strand RNA of chain II

<400> 1
gguauaugcu gugugua 17

<210> 2
<211> 16
<212> RNA
<213> Artificial sequence

<220>
<223> single strand RNA of chain III

<400> 2
agcagaguac acacag 16

<210> 3
<211> 24
<212> RNA
<213> Artificial sequence

<220>
<223> a part of ligated single strand RNA

<400> 3
agcagaguac acacagcaua uacc 24

<210> 4
<211> 26
<212> RNA
<213> Artificial sequence

<220>
<223> a part of ligated single strand RNA

<400> 4
gguauaugcu guguguacuc ugcuuc 26

<210> 5
<211> 12
<212> RNA
<213> Artificial sequence

<220>
<223> a part of single strand RNA of chain IV

<400> 5
guacucugcu uc 12

<210> 6
<211> 14
<212> RNA
<213> Artificial sequence

<220>
<223> a part of single strand RNA of chain V

<400> 6
gguauaugcu gugu 14

<210> 7
<211> 20
<212> RNA
<213> Artificial sequence

<220>
<223> a part of single strand RNA of chain VI

<400> 7
agcagaguac acacagcaua 20

<210> 8
<211> 22
<212> RNA
<213> Artificial sequence

<220>
<223> single strand RNA of chain X

<400> 8
agcagaguac acacagcaua ua 22

<210> 9
<211> 23
<212> RNA
<213> Artificial sequence

<220>
<223> single strand RNA of chain XII

<400> 9
agcagaguac acacagcaua uac 23

<210> 10
<211> 334
<212> PRT

<213>  bacteriophage T4

<400>  10

Met Phe Lys Lys Tyr Ser Ser Leu Glu Asn His Tyr Asn Ser Lys Phe
1               5                   10                  15

Ile Glu Lys Leu Tyr Ser Leu Gly Leu Thr Gly Gly Glu Trp Val Ala
            20                  25                  30

Arg Glu Lys Ile His Gly Thr Asn Phe Ser Leu Ile Ile Glu Arg Asp
        35                  40                  45

Lys Val Thr Cys Ala Lys Arg Thr Gly Pro Ile Leu Pro Ala Glu Asp
    50                  55                  60

Phe Phe Gly Tyr Glu Ile Ile Leu Lys Asn Tyr Ala Asp Ser Ile Lys
65                  70                  75                  80

Ala Val Gln Asp Ile Met Glu Thr Ser Ala Val Val Ser Tyr Gln Val
                85                  90                  95

Phe Gly Glu Phe Ala Gly Pro Gly Ile Gln Lys Asn Val Asp Tyr Cys
            100                 105                 110

Asp Lys Asp Phe Tyr Val Phe Asp Ile Ile Val Thr Thr Glu Ser Gly
        115                 120                 125

Asp Val Thr Tyr Val Asp Asp Tyr Met Met Glu Ser Phe Cys Asn Thr
    130                 135                 140

Phe Lys Phe Lys Met Ala Pro Leu Leu Gly Arg Gly Lys Phe Glu Glu
145                 150                 155                 160

Leu Ile Lys Leu Pro Asn Asp Leu Asp Ser Val Val Gln Asp Tyr Asn
                165                 170                 175

Phe Thr Val Asp His Ala Gly Leu Val Asp Ala Asn Lys Cys Val Trp
            180                 185                 190

Asn Ala Glu Ala Lys Gly Glu Val Phe Thr Ala Glu Gly Tyr Val Leu
        195                 200                 205

Lys Pro Cys Tyr Pro Ser Trp Leu Arg Asn Gly Asn Arg Val Ala Ile
    210                 215                 220

Lys Cys Lys Asn Ser Lys Phe Ser Glu Lys Lys Lys Ser Asp Lys Pro
225                 230                 235                 240

Ile Lys Ala Lys Val Glu Leu Ser Glu Ala Asp Asn Lys Leu Val Gly
245 250 255

Ile Leu Ala Cys Tyr Val Thr Leu Asn Arg Val Asn Asn Val Ile Ser
260 265 270

Lys Ile Gly Glu Ile Gly Pro Lys Asp Phe Gly Lys Val Met Gly Leu
275 280 285

Thr Val Gln Asp Ile Leu Glu Glu Thr Ser Arg Glu Gly Ile Thr Leu
290 295 300

Thr Gln Ala Asp Asn Pro Ser Leu Ile Lys Lys Glu Leu Val Lys Met
305 310 315 320

Val Gln Asp Val Leu Arg Pro Ala Trp Ile Glu Leu Val Ser
325 330

<210> 11
<211> 335
<212> PRT
<213> Vibrio phage KVP40

<400> 11

Met Ser Phe Val Lys Tyr Thr Ser Leu Glu Asn Ser Tyr Arg Gln Ala
1 5 10 15

Phe Val Asp Lys Cys Asp Met Leu Gly Val Arg Glu Trp Val Ala Leu
20 25 30

Glu Lys Ile His Gly Ala Asn Phe Ser Phe Ile Val Glu Phe Lys Pro
35 40 45

Asn Glu Ala Gln Asp Gly Ala Glu Phe Thr Val Thr Pro Ala Lys Arg
50 55 60

Thr Ser Thr Ile Gly Ala Asn Val Met Gly Asp Tyr Asp Phe Tyr Gly
65 70 75 80

Cys Thr Ser Val Val Glu Ala His Thr Ala Lys Met Glu Ala Ile Ser
85 90 95

Asn Trp Leu Trp Ala Arg Gly Ile Ile Asn Val Gly Glu Thr Ile Ile
100 105 110

Val Tyr Gly Glu Leu Ala Gly Lys Gly Val Gln Lys Glu Val Asn Tyr
115 120 125

Gly Asp Lys Asp Phe Trp Ala Tyr Asp Ile Leu Leu Pro Glu Thr Gly
130 135 140

Lys Phe Leu Asp Trp Asp Val Val Leu Glu Ala Cys Glu Phe Ala Lys
145 150 155 160

Val Lys Thr Thr His Glu Ile Ala Arg Gly Thr Leu Asp Glu Leu Leu
165 170 175

Arg Ile Asp Pro Leu Phe Arg Ser Phe His Thr Pro Ala Asp Val Asp
180 185 190

Gly Asp Asn Val Ala Glu Gly Phe Val Val Lys Gln Leu Arg Asn Glu
195 200 205

Lys Arg Leu His Asn Gly Ser Arg Ala Ile Leu Lys Val Lys Asn Asp
210 215 220

Lys Phe Lys Glu Lys Lys Asn Lys Ala Gly Lys Thr Pro Arg Ala Ala
225 230 235 240

Val Val Leu Thr Glu Glu Gln Glu Lys Leu His Ala Ala Phe Ser Cys
245 250 255

Tyr Leu Thr Glu Asn Arg Leu Arg Asn Val Leu Ser Lys Leu Glu Thr
260 265 270

Val Thr Gln Lys Gln Phe Gly Met Ile Ser Gly Leu Phe Ile Lys Asp
275 280 285

Ala Lys Asp Glu Phe Glu Arg Asp Glu Leu Asn Glu Thr Ala Ile Ala
290 295 300

Arg Asp Asp Trp Asp Val Ile Lys Arg Ser Leu Thr Asn Ile Ala Asn
305 310 315 320

Glu Ile Leu Arg Lys Asn Trp Leu Asp Ile Leu Asp Gly Asn Phe
325 330 335

<210> 12
<211> 342
<212> PRT
<213> Deinococcus radiorurans

<400> 12

Met Ala Glu Arg Gln Val Ile Lys Glu Arg Ala Gln Leu Phe Pro His
1 5 10 15

Pro Asn Ala Glu Arg Leu Glu Leu Cys Lys Val Gly Thr Phe Gln Leu
20 25 30

Val Val Arg Lys Gly Glu Tyr Arg Asp Gly Asp Pro Ile Val Ile Ala
35 40 45

Pro Glu Lys Ala Val Leu Pro Pro Gln Leu Ala Gly Leu Tyr Thr Asn
50 55 60

Ala Asp Thr Gly Ala Ser Tyr Leu His Gly Ala Glu Lys Asn Arg Val
65 70 75 80

Gly Ser Val Arg Leu Arg Gly Glu Val Ser Gln Gly Val Ile Leu Pro
85 90 95

Leu Asp Gly Leu Glu Asp Ala Pro Phe Gly Glu Asp Leu Ala Glu Arg
100 105 110

Leu Gly Ile Thr Phe Trp Glu Pro Pro Val Pro Val Ser Met Ala Gly
115 120 125

Glu Val Glu Pro Arg Pro Pro Ala Gln His Tyr Lys His His Asp Val
130 135 140

Glu Gln Phe Gly Ile Tyr Val Ser Glu Phe Ala Ser Gly Glu Glu Val
145 150 155 160

Met Val Thr Glu Lys Leu His Gly Thr Gln Gly Val Tyr Phe Arg Thr
165 170 175

Ala Glu Gly Arg Trp Leu Val Thr Ser Lys Gly Leu Ser Arg Gly Gly
180 185 190

Leu Thr Leu Arg Glu Ala Ala Ser Asn Val Tyr Trp Gln Ala Ala Arg
195 200 205

Asn Ser Asn Leu Phe Ala Glu Ala Asp Ala Ala Phe Ser Gly Gly Glu
210 215 220

Val Gln Ile Phe Gly Glu Val Val Pro Val Gln Lys Gly Phe Ser Tyr
225 230 235 240

Gly Gln His Lys Pro Thr Val Phe Val Phe Lys Val Val Tyr Asp Gly
245 250 255

Leu Arg Leu Pro Arg Arg Asp Trp Pro Gln Trp Val Leu Asp His Ala
260 265 270

```
Val Pro Val Leu Tyr Glu Gly Pro Phe Asp Glu Ala Thr Val Arg Lys
        275                 280                 285

Leu Arg Gly Gly Leu Glu Thr Val Ser Gly Lys Gly Leu His Ile Arg
    290                 295                 300

Glu Gly Val Val Val Ala Pro Lys Val Pro Arg Phe Ala Ala Asp Gly
305                 310                 315                 320

Ser Asp Leu Ser Val Lys Leu Ile Ser Asp Ala Tyr Ala Lys Lys Glu
                325                 330                 335

Thr Gly Glu Glu Tyr Ser
            340
```